# EUROPEAN PATENT APPLICATION

(11) **EP 2 387 998 A1**
(43) Date of publication of application: **23.11.2011**
(21) Application number: 11158237.5
(22) Date of filing: 29.04.2004
(51) Int. Cl.: A61K 31/415, A61P 43/00, A61P 25/28, A61P 9/10, A61K 31/4152

(54) **Methods for treating degenerative diseases/injuries**

(30) Priority: 29.04.2003 US 466540 P; 19.05.2003 US 471554 P; 14.08.2003 US 495034 P; 04.03.2004 US 549977 P; 19.03.2004 US 554581 P; 25.03.2004 US 556390 P
(62) Divisional of application: 04760459.0
(71) Applicant: Glaxosmithkline LLC, Philadelphia, PA 19102 (US)
(72) Inventor: Erickson-Miller, Connie L, Collegeville, PA 19426 (US); Jenkins, Julian, Collegeville, PA 19426 (US)
(74) Representative: Learoyd, Stephanie Anne

(57) **Abstract**

Invented is a method of treating degenerative diseases/injuries, in a mammal, including a human, in need thereof which comprises the administration of a therapeutically effective amount of a non-peptide TPO receptor agonist to such mammal.

## Description

### FIELD OF THE INVENTION

This invention relates to non-peptide thrombopoietin (TPO ) receptor agonists and their use in the treatment of degenerative diseases/injuries.

### BACKGROUND OF THE INVENTION

Thrombopoietin (TPO) has been shown to be the main humoral regulator in situations involving thrombocytopenia. See, e.g., Metcalf Nature 369:519-520 (1994). TPO has been shown in several studies to increase platelet counts, increase platelet size, and increase isotope incorporation into platelets of recipient animals. Because platelets (thrombocytes) are necessary for blood clotting and when their numbers are very low a patient is at risk of death from catastrophic hemorrhage, TPO is considered to have potential useful applications in both the diagnosis and the treatment of various hematological disorders, for example, diseases primarily due to platelet defects. In addition, studies have provided a basis for the projection of efficacy of TPO therapy in the treatment of thrombocytopenia, and particularly thrombocytopenia resulting from chemotherapy, radiation therapy, or bone marrow transplantation as treatment for cancer or lymphoma. See e.g., McDonald (1992) Am. J. Ped. Hematology/Oncology 14: 8-21 (1992).
The slow recovery of platelet levels in patients suffering from thrombocytopenia is a serious problem, and has lead to the search for small molecule non-peptide TPO receptor agonists that are able to accelerate platelet regeneration. (e.g. see, International Application Number PCT/US01/16863, having International Filing Date May 24, 2001).
However, non-peptide TPO receptor agonists are not known to have a beneficial effect in the treatment of degenerative diseases/injuries.
It would be desirable to provide compounds which allow for the treatment of degenerative diseases/injuries.

The present invention relates to novel therapeutic uses of a known class of compounds, non-peptide TPO receptor agonists. The present invention concerns a method for treating degenerative diseases/injuries in a mammal in need of such treatment.

As disclosed herein it has unexpectedly been discovered that non-peptide TPO receptor agonist compounds are useful in treating degenerative diseases/injuries.

As disclosed herein it has unexpectedly been discovered that the in vivo administration of a non-peptide TPO receptor agonist is useful in treating degenerative diseases/injuries.

As disclosed herein it has unexpectedly been discovered that non-peptide TPO receptor agonists increase the survival of stem cells to a therapeutic extent.

As disclosed herein it has unexpectedly been discovered that non-peptide TPO receptor agonists stimulate the production of stem cells to a therapeutic extent.

As disclosed herein it has unexpectedly been discovered that non-peptide TPO receptor agonists increase the number of stem cells to a therapeutic extent.

As disclosed herein it has unexpectedly been discovered that non-peptide TPO receptor agonists increase stem cell longevity to a therapeutic extent.

As disclosed herein it has unexpectedly been discovered that the in vivo administration of a non-peptide TPO receptor agonist increases the survival of stem cells to a therapeutic extent.

As disclosed herein it has unexpectedly been discovered that the in vivo administration of a non-peptide TPO receptor agonist stimulates the production of stem cells to a therapeutic extent.

As disclosed herein it has unexpectedly been discovered that the in vivo administration of a non-peptide TPO receptor agonist increases stem cell function to a therapeutic extent.

As disclosed herein it has unexpectedly been discovered that the in vivo administration of a non-peptide TPO receptor agonist increases stem cell longevity to a therapeutic extent.

### SUMMARY OF THE INVENTION

This invention relates to a method of treating a degenerative disease/injury in a mammal, including a human, in need thereof which comprises administering to such mammal a therapeutically effective amount of a non-peptide TPO receptor agonists.

This invention also relates to the discovery that non-peptide TPO receptor agonists are effective in the treatment of degenerative diseases/injuries.

This invention also relates to the discovery that non-peptide TPO receptor agonists increase the survival of stem cells to a therapeutic extent.

This invention also relates to the discovery that non-peptide TPO receptor agonists stimulate the production of stem cells to a therapeutic extent.

This invention also relates to the discovery that non-peptide TPO receptor agonists increase the number of stem cells to a therapeutic extent.

This invention also relates to the discovery that non-peptide TPO receptor agonists increase stem cell longevity to a therapeutic extent.

This invention also relates to the discovery that the in vivo administration of a non-peptide TPO receptor agonist increases the survival of stem cells to a therapeutic extent.

This invention also relates to the discovery that the in vivo administration of a non-peptide TPO receptor agonist stimulates the production of stem cells to a therapeutic extent.

This invention also relates to the discovery that the in vivo administration of a non-peptide TPO receptor agonist increases stem cell function to a therapeutic extent.

This invention also relates to the discovery that the in vivo administration of a non-peptide TPO receptor agonist increases stem cell longevity to a therapeutic extent.

Included among the non-peptide TPO receptor agonists of the invention are compounds of Formula (I): 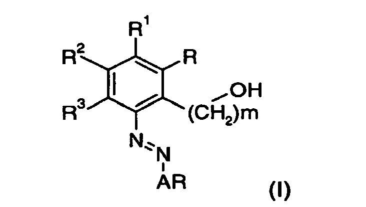 wherein:
R, R¹, R² and R³ are each independently selected from hydrogen, C₁₋₆alkyl, -(CH₂)ₚOR⁴, -C(O)OR⁴, formyl, nitro, cyano, halogen, aryl, substituted aryl, substituted alkyl, -S(O)ₙR⁴, cycloalkyl, -NR⁵R⁶, protected -OH, -CONR⁵R⁶, phosphonic acid, sulfonic acid, phosphinic acid, -SO₂NR⁵R⁶, and a heterocyclic methylene substituent as represented by Formula (III), 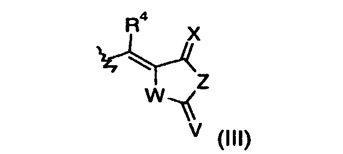 where,
   p is 0-6,
   n is 0-2,
   V, W, X and Z are each independently selected from O, S and NR¹⁶, where R¹⁶ is selected from: hydrogen, alkyl, cycloalkyl, C₁-C₁₂aryl, substituted alkyl, substituted cycloalkyl and substituted C₁-C₁₂aryl, R⁴ is selected from: hydrogen, alkyl, cycloalkyl, C₁-C₁₂aryl, substituted alkyl, substituted cycloalkyl and substituted C₁-C₁₂aryl, and R⁵ and R⁶ are each independently selected from hydrogen, alkyl, substituted alkyl, C₃₋₆cycloalkyl, and aryl,
   or R⁵ and R⁶ taken together with the nitrogen to which they are attached represent a 5 to 6 member saturated ring containing up to one other heteroatom selected from oxygen and nitrogen;
   m is 0-6; and
   AR is a cyclic or polycyclic aromatic ring containing from 3 to 16 carbon atoms and optionally containing one or more heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, substituted alkyl, aryl, substituted cycloalkyl, substituted aryl, aryloxy, oxo, hydroxy, alkoxy, cycloalkyl, acyloxy, amino, N-acylamino, nitro, cyano, halogen, -C(O)OR⁴, -C(O)NR¹⁰R¹¹, -S(O)₂NR¹⁰R¹¹, -S(O)ₙR⁴ and protected -OH,
   where n is 0-2,
   R⁴ is hydrogen, alkyl, cycloalkyl, C₁-C₁₂aryl, substituted alkyl, substituted cycloalkyl and substituted C₁-C₁₂aryl, and
   R¹⁰ and R¹¹ are independently hydrogen, cycloalkyl, C₁-C₁₂aryl, substituted cycloalkyl, substituted C₁-C₁₂aryl, alkyl or alkyl substituted with one or more substituents selected from the group consisting of: alkoxy, acyloxy, aryloxy, amino, N-acylamino, oxo, hydroxy, -C(O)OR⁴, - S(O)ₙR⁴, -C(O)NR⁴R⁴, -S(O)₂NR⁴R⁴, nitro, cyano, cycloalkyl, substituted cycloalkyl, halogen, aryl, substituted aryl and protected -OH, or R¹⁰ and R¹¹ taken together with the nitrogen to which they are attached represent a 5 to 6 member saturated ring containing up to one other heteroatom selected from oxygen and nitrogen, where R⁴ is as described above and n is 0-2;
   and/or pharmaceutically acceptable salts, hydrates, solvates and esters thereof;
   provided that at least one of R, R¹, R² and R³ is a substituted aryl group or a heterocyclic methylene substituent as represented in Formula (III).

This invention relates to a method of treating degenerative diseases/injuries, which comprises administering to a subject in need thereof a therapeutically effective amount of a non-peptide TPO receptor agonist of Formula (I).

Included in the present invention are pharmaceutical compositions comprising a pharmaceutical carrier and compounds useful in the methods of the invention.

Also included in the present invention are methods of co-administering non-peptide TPO receptor agonists with further active ingredients.

### DETAILED DESCRIPTION OF THE INVENTION

This invention relates to methods of treating a degenerative disease/injury in a mammal, including a human, in need thereof which comprises administering to such mammal a therapeutically effective amount of a non-peptide TPO receptor agonist, including compounds of Formula (I) as described above.

Included among the compounds that are useful in the present invention are those having Formula (V): 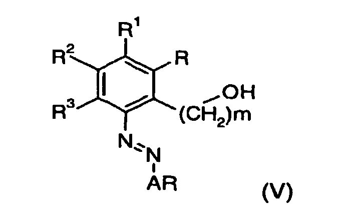 wherein:
R, R¹, R² and R³ are each independently selected from hydrogen, C₁₋₆alkyl, C₁₋₆alkoxy, -(CH₂)ₚOR⁴, -C(O)OR⁴, formyl, nitro, cyano, halogen, aryl, substituted aryl, substituted alkyl, -S(O)ₙR⁴, cycloalkyl, -NR⁵R⁶, protected -OH, -CONR⁵R⁶, phosphonic acid, sulfonic acid, phosphinic acid and -SO₂NR⁵R⁶,
   where,
   p is 0-6,
   n is 0-2,
   R⁴ is selected from: hydrogen, alkyl, cycloalkyl, C₁-C₁₂aryl, substituted alkyl, substituted cycloalkyl and substituted C₁-C₁₂aryl, and R⁵ and R⁶ are each independently selected from hydrogen, alkyl, substituted alkyl, C₃₋₆cycloalkyl, and aryl, or R⁵ and R⁶ taken together with the nitrogen to which they are attached represent a 5 to 6 member saturated ring containing up to one other heteroatom selected from oxygen and nitrogen;
m is 0-6; and
AR is a cyclic or polycyclic aromatic ring containing from 3 to 16 carbon atoms and optionally containing one or more heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, substituted alkyl, aryl, substituted cycloalkyl, substituted aryl, aryloxy, oxo, hydroxy, alkoxy, cycloalkyl, acyloxy, amino, N-acylamino, nitro, cyano, halogen, -C(O)OR⁴, -C(O)NR¹⁰R¹¹, -S(O)₂NR¹⁰R¹¹, -S(O)ₙR⁴ and protected -OH,
   where n is 0-2,
R⁴ is hydrogen, alkyl, cycloalkyl, C₁-C₁₂aryl, substituted alkyl, substituted cycloalkyl and substituted C₁-C₁₂aryl; and R¹⁰ and R¹¹ are independently hydrogen, cycloalkyl, C₁-C₁₂aryl, substituted cycloalkyl, substituted C₁-C₁₂aryl, alkyl or alkyl substituted with one or more substituents selected from the group consisting of: alkoxy, acyloxy, aryloxy, amino, N-acylamino, oxo, hydroxy, -C(O)OR⁴, - S(O)ₙR⁴, -C(O)NR⁴R⁴, -S(O)₂NR⁴R⁴, nitro, cyano, cycloalkyl, substituted cycloalkyl, halogen, aryl, substituted aryl and protected -OH, or R¹⁰ and R¹¹ taken together with the nitrogen to which they are attached represent a 5 to 6 member saturated ring containing up to one other heteroatom selected from oxygen and nitrogen,
   where R⁴ is as described above and n is 0-2;
and/or pharmaceutically acceptable salts, hydrates, solvates and esters thereof;
provided that at least one of R, R¹, R² and R³ is a substituted aryl group.

Included among the compounds that are useful in the present invention are those having Formula (II): 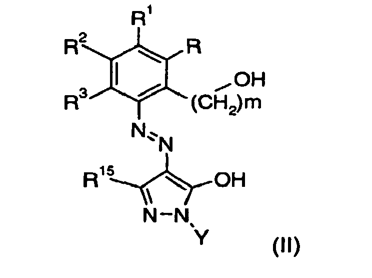 wherein:
R, R¹, R² and R³ are each independently selected from hydrogen, C₁₋₆alkyl, -(CH₂)pOR⁴, -C(O)OR⁴, formyl, nitro, cyano, halogen, aryl, substituted aryl, substituted alkyl, -S(O)ₙR⁴, cycloalkyl, -NR⁵R⁶, protected -OH, -CONR⁵R⁶, phosphonic acid, sulfonic acid, phosphinic acid, -SO₂NR⁵R⁶, and a heterocyclic methylene substituent as represented by Formula (III), 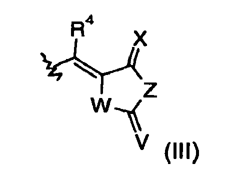 where
   p is 0-6,
   n is 0-2,
   V, W, X and Z are each independently selected from O, S, and NR¹⁶, where R¹⁶ is selected from: hydrogen, alkyl, cycloalkyl, C₁-C₁₂aryl, substituted alkyl, substituted cycloalkyl and substituted C₁-C₁₂aryl,
   R⁴ is hydrogen, alkyl, cycloalkyl, C₁-C₁₂aryl, substituted alkyl, substituted cycloalkyl and substituted C₁-C₁₂aryl, and
   R⁵ and R⁶ are each independently selected from hydrogen, alkyl, substituted alkyl, C₃-₆cycloalkyl, and aryl,
   or R⁵ and R⁶ taken together with the nitrogen to which they are attached represent a 5 to 6 member saturated ring containing up to one other heteroatom selected from oxygen and nitrogen;
R¹⁵ is selected from the group consisting of alkyl, C₁-C₁₂aryl, hydroxy, alkoxy, substituted alkyl, substituted C₁-C₁₂aryl and halogen;
m is 0-6; and
Y is selected from alkyl, substituted alkyl and a cyclic or polycyclic aromatic ring containing from 3 to 14 carbon atoms and optionally containing from one to three heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, substituted alkyl, C₁-C₁₂aryl, substituted cycloalkyl, substituted C₁-C₁₂aryl, hydroxy, aryloxy, alkoxy, cycloalkyl, nitro, cyano, halogen and protected -OH;
and/or pharmaceutically acceptable salts, hydrates, solvates and esters thereof;
provided that at least one of R, R¹, R² and R³ is a substituted aryl group or a heterocyclic methylene substituent as represented in Formula (III).

Included among compounds of Formula (II) that are useful in the current invention are those having Formula (VI): 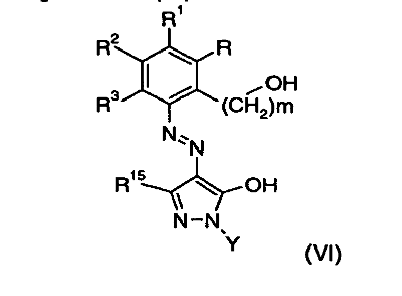 wherein:
R, R¹, R² and R³ are each independently selected from hydrogen, C₁₋₆alkyl, C₁₋₆alkoxy, -(CH₂)ₚOR⁴, -C(O)OR⁴, formyl, nitro, cyano, halogen, aryl, substituted aryl, substituted alkyl, -S(O)ₙR⁴, cycloalkyl, -NR⁵R⁶, protected -OH, -CONR⁵R⁶, phosphonic acid, sulfonic acid, phosphinic acid and -SO₂NR⁵R⁶,
   where
   p is 0-6,
   n is 0-2,
   R⁴ is hydrogen, alkyl, cycloalkyl, C₁-C₁₂aryl, substituted alkyl, substituted cycloalkyl and substituted C₁-C₁₂aryl, and R⁵ and R⁶ are each independently selected from hydrogen, alkyl, substituted alkyl, C₃₋₆cycloalkyl, and aryl,
   or R⁵ and R⁶ taken together with the nitrogen to which they are attached represent a 5 to 6 member saturated ring containing up to one other heteroatom selected from oxygen and nitrogen;
R¹⁵ is selected from the group consisting of alkyl, C₁-C₁₂aryl, hydroxy, alkoxy, substituted alkyl, substituted C₁-C₁₂aryl and halogen;
m is 0-6; and
Y is selected from alkyl, substituted alkyl and a cyclic or polycyclic aromatic ring containing from 3 to 14 carbon atoms and optionally containing from one to three heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, substituted alkyl, C₁-C₁₂aryl, substituted cycloalkyl, substituted C₁-C₁₂aryl, hydroxy, aryloxy, alkoxy, cycloalkyl, nitro, cyano, halogen and protected -OH;
and pharmaceutically acceptable salts, hydrates, solvates and esters thereof;
provided that at least one of R, R¹, R² and R³ is a substituted aryl group.

Included among the compounds useful in the present invention are those having Formula (VI) in which, either:
R is a substituted aryl; and R¹ is hydrogen;
   or:
R is hydrogen; and R¹ is a substituted aryl;
   and in either case:
R² and R³ are each independently selected from hydrogen, C₁₋₆alkyl, C₁₋₆alkoxy, nitro, cyano, halogen, aryl, substituted aryl, substituted alkyl, cycloalkyl, phosphonic acid, phosphinic acid and sulfonic acid;
R¹⁵ is selected from the group consisting of alkyl, substituted alkyl, C₁-C₁₂aryl, alkoxy and halogen;
m is 0-4; and
Y is selected from, phenyl, pyridinyl and pyrimidinyl, where the phenyl, pyridinyl and pyrimidinyl are optionally substituted with from one to three substituents selected from the group consisting of: alkyl, substituted alkyl, C₁-C₁₂aryl, substituted C₁-C₁₂aryl, alkoxy and halogen;
and pharmaceutically acceptable salts, hydrates, solvates and esters thereof.

Included among the compounds useful in the present invention are those having Formula (VI) in which, -
R is a substituted C₁-C₁₂aryl; and
R¹ is hydrogen;
R² and R³ are each independently selected from hydrogen, C₁-₆alkyl, C₁₋₆alkoxy, nitro, cyano, halogen, substituted alkyl and cycloalkyl;
R¹⁵ is selected from the group consisting of alkyl, substituted alkyl, C₁-C₁₂aryl, alkoxy and halogen;
m is 0-2; and
Y is selected from,
   phenyl, pyridinyl and pyrimidinyl, where the phenyl, pyridinyl and pyrimidinyl are optionally substituted with from one to three substituents selected from the group consisting of: alkyl, substituted alkyl, C₁-C₁₂aryl, substituted C₁-C₁₂aryl, alkoxy and halogen;
and pharmaceutically acceptable salts, hydrates, solvates and esters thereof.

Included among the compounds useful in the present invention are those having Formula (VI) in which,
R is a substituted phenyl or pyridinyl ring; and
R¹ is hydrogen;
R² and R³ are each independently selected from hydrogen, C₁₋₆alkyl, substituted alkyl and halogen;
R¹⁵ is selected from the group consisting of C₁₋₄alkyl, C₁₋₄alkoxy, C₁-C₁₂aryl and halogen;
m is 0; and
Y is selected from,
   phenyl, pyridinyl and pyrimidinyl, where the phenyl, pyridinyl and pyrimidinyl is optionally substituted with from one to three substituents selected from the group consisting of: alkyl, substituted alkyl, C₁-C₁₂aryl, substituted C₁₋C₁₂aryl, alkoxy and halogen;
and pharmaceutically acceptable salts, hydrates, solvates and esters thereof.

Included among the compounds useful in the present invention are:
4'-{N'-[1-(3,4-Dimethylphenyl)-3-methyl-5-oxo-1,5-dihydropyrazol-4-ylidene]hydrazino}-3'-hydroxybiphenyl-4-carboxylic acid;
4'-{N'-[1-(3,4-Dimethylphenyl)-3-methyl-5-oxo-1,5-dihydropyrazol-4-ylidene]hydrazino}-3'-hydroxybiphenyl-3-carboxylic acid;
₃'-{N'-[1-(3,4-Dimethylphenyl)-3-methyl-5-oxo-1,5-dihydropyrazol-4-ylidene]hydrazino}-2'-hydroxybiphenyl-3-carboxylic acid;
3'-{N'-[1-(4-tert-Butylphenyl)-3-methyl-5-oxo-1,5-dihydropyrazol-4-ylidene]hydrazino}-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-5'-chloro-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-2'-hydroxybiphenyl-3-carboxylic acid;

3-Aza-3'-{N'-[1-(4-tert-butylphenyl)-3-methyl-5-oxo-1,5-dihydropyrazol-4-ylidene]hydrazino}-2'-hydroxybiphenyl-5-carboxylic acid;
2-Aza-5'-chloro-3'-{N'-[1-(3,4-dimethylphenyl)-3-methyl-5-oxo-1,5-dihydropyrazol-4-ylidene]hydrazino}-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-2'-hydroxy-5'-methylbiphenyl-3-carboxylic acid;
2-Aza-3'-{N'-[1-(3,4-dimethylphenyl)-3-methyl-5-oxo-1,5-dihydropyrazol-4-ylidene]hydrazino}-2'-hydroxy-5'-methylbiphenyl-3-carboxylic acid;
3'-{N'-[1-(4-tert-Butylphenyl)-3-methyl-5-oxo-1,5-dihydropyrazol-4-ylidene]hydrazino}-2'-hydroxy-5'-methylbiphenyl-3-carboxylic acid;
3-{N'-[1-(3,4-dimethylphenyl)-3-methyl-5-oxo-1,5-dihydropyrazol-4-ylidene]hydrazino}-2-hydroxy-3'-(tetrazol-5-yl)biphenyl;
3'-{N'-[1-(3,4-dimethylphenyl)-3-methyl-5-oxo-1,5-dihydropyrazol-4-ylidene]hydrazino}-5'-fluoro-2'-hydroxybiphenyl-3-carboxylic acid;
7-({N'-[1-(3,4-dimethylphenyl)-3-methyl-5-oxo-1,5-dihydropyrazol-4-ylidene]hydrazino}-2-hydroxyphenyl)quinolin-4[1H]-one-3-carboxylic acid;
7-({N'-[1-(4-tert-butylphenyl)-3-methyl-5-oxo-1,5-dihydropyrazol-4-ylidene]hydrazino}-2-hydroxyphenyl)quinolin-4[1H]-one-3-carboxylic acid;
3-Aza-3'-{N'-[1-(3,4-dimethylphenyl)-3-methyl-5-oxo-1,5-dihydropyrazol-4-ylidene]hydrazino}-2'-hydroxybiphenyl-5-carboxylic acid;
3-Aza-3'-(N'-[1-{3-methyl-[4-(1-methylethyl)phenyl]-5-oxo-1,5-dihydropyrazol-4-ylidene}hydrazino)-2'-hydroxybiphenyl-5-carboxylic acid;
3-Aza-3'-{N'-[1-(4-tertbutylphenyl-3-methyl-5-oxo-1,5-dihydropyrazol-4-ylidene]hydrazino}-2'-hydroxybiphenyl-5-carboxylic acid;
5'-Chloro-3'-{N'-[1-(3,4-dimethylphenyl)-3-methyl-5-oxo-1,5-dihydropyrazol-4-ylidene]hydrazino}-2'-hydroxybiphenyl-3-carboxylic acid;
3'-{N'-[1-(3,4-Dimethylphenyl)-3,5-dioxo-1,5-dihydropyrazol-4-ylidene]hydrazino}-2'-hydroxybiphenyl-3-carboxylic acid;
3'-{N'-[1-(2-Ethoxy-2-oxoethyl)-3-methyl-5-oxo-1,5-dihydropyrazol-4-ylidene]hydrazino}-2'-hydroxybiphenyl-3-carboxylic acid;
3-{N'-[1-(3,4-dimethylphenyl)-3-methyl-5-oxo-1,5-dihydropyrazol-4-ylidene]hydrazino}-2-hydroxy-4'-(tetrazol-5-yl)biphenyl;
3'-(N'-{1-[2-(N-tert-butyl)amino-2-oxoethyl]-3-methyl-5-oxo-1,5-dihydropyrazol-4-ylidene}hydrazino)-2'-hydroxybiphenyl-3-carboxylic acid;
3'-{N'-[3-Chloro-1-(3,4-dimethylphenyl)-5-oxo-1,5-dihydropyrazol-4-ylidene]hydrazino}-2'-hydroxybiphenyl-3-carboxylic acid;
5-chloro-3-{N'-[1-(3,4-dimethylphenyl)-3-methyl-5-oxo-1,5-dihydropyrazol-4-ylidene]hydrazino}-2-hydroxy-4'-(tetrazol-5-yl)biphenyl;
3'-{N'-[1-(3,4-Dimethylphenyl)-3-methyl-5-oxo-1,5-dihydropyrazol-4-ylidene]hydrazino}-2'-hydroxybiphenyl-3,5-dicarboxylic acid;
3-Aza-3'-{N'-[1-(3,4-dimethylphenyl)-3-methyl-5-oxo-1,5-dihydropyrazol-4-ylidene]hydrazino}-2'-hydroxy-5'-methylbiphenyl-5-carboxylic acid;
3'-{N'-[1-(3,4-Dimethylphenyl)-3-methyl-5-oxo-1,5-dihydropyrazol-4-ylidene]hydrazino}-2'-hydroxybiphenyl-4-carboxylic acid;
3'-{N'-[1-(3,4-Dimethylphenyl)-3-methoxy-5-oxo-1,5-dihydropyrazol-4-ylidene]hydrazino}-2'-hydroxybiphenyl-3-carboxylic acid;
3'-{N'-[1-(4-methoxyphenyl)-3-methyl-5-oxo-1,5-dihydropyrazol-4-ylidene]hydrazino}-2'-hydroxybiphenyl-3-carboxylic acid;
(3-{N'-[1-(3,4-dimethylphenyl)-3-methyl-5-oxo-1,5-dihydropyrazol-4-ylidene]hydrazino}-2-hydroxy-3'-biphenyl)-1,1,1,-trifluoromethanesulfonamide;
3'-{N'-[1-(3,4-Dichlorophenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(3-trifluoromethylphenyl)-1,5-dihydropyrazol-4-ylidene]hydrazino}-2'-hydroxybiphenyl-3-carboxylic acid;
8-{N'-[1-(3,4-dimethylphenyl)-3-methyl-5-oxo-1,5-dihydropyrazol-4-ylidene]hydrazino}quinolin-4[1H]-one-3-carboxylic acid;
3'-{N'-[3-methyl-5-oxo-1-(4-trifluoromethylphenyl)-1,5-dihydropyrazol-4-ylidene]hydrazino}-2'-hydroxybiphenyl-3-carboxylic acid;
3'-{N'-[3-methyl-5-oxo-1-(4-N-methylcarboxamidolphenyl)-1,5-dihydropyrazol-4-ylidene]hydrazino}-2'-hydroxybiphenyl-3-carboxylic acid;
N-[1-(3'-{N'-[1-(3,4-dimethylphenyl)-3-methyl-5-oxo-1,5-dihydropyrazol-4-ylidene]hydrazino}-2'-hydroxybiphenyl-3-yl)methanoyl]methanesulfonamide;
3'-{N'-[3-methyl-5-oxo-1-phenyl-1,5-dihydropyrazol-4-ylidene]hydrazino}-2'-hydroxybiphenyl-3-carboxylic acid;
3'-{N'-[3-methyl-1-(4-methylphenyl)-5-oxo-1,5-dihydropyrazol-4-ylidene]hydrazino}-2'-hydroxybiphenyl-3-carboxylic acid;
3'-{N'-[1-(4-chlorophenyl)-3-methyl-5-oxo-1,5-dihydropyrazol-4-ylidene]hydrazino}-2'-hydroxybiphenyl-3-carboxylic acid;
3'-{N'-[1-(4-fluorophenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(4-trifluoromethoxyphenyl)-1,5-dihydropyrazol-4-ylidene]hydrazino}-2'-hydroxybiphenyl-3-carboxylic acid;
3'-{N'-[1-(3,4-dimethylphenyl)-3-ethoxy-5-oxo-1,5-dihydropyrazol-4-ylidene]hydrazino}-2'-hydroxybiphenyl-3-carboxylic acid;
3'-{N'-[1-(3,4-dimethylphenyl)-3-(1-methylethoxy)-5-oxo-1,5-dihydropyrazol-4-ylidene]hydrazino}-2'-hydroxybiphenyl-3-carboxylic acid;
3'-{N'-[3-tert-butyl-1-(3,4-dimethylphenyl)-5-oxo-1,5-dihydropyrazol-4-ylidene]hydrazino}-2'-hydroxybiphenyl-3-carboxylic acid;
3'-{N'-[3-methyl-1-(4-methyl-2,3,5,6-tetrafluorophenyl)-5-oxo-1,5-dihydropyrazol-4-ylidene]hydrazino}-2'-hydroxybiphenyl-3-carboxylic acid;
3'-{N'-[1-(4-fluoro-3-methylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-phenyl-5-oxo-1,5-dihydropyrazol-4-ylidene]hydrazino}-2'-hydroxybiphenyl-3-carboxylic acid;
3-{N'-[1-(3,4-dimethylphenyl)-5-oxo-3-phenyl-1,5-dihydropyrazol-4-ylidene]hydrazino}-2-hydroxy-3'-tetrazol-5-ylbiphenyl;
3-{N'-[1-(3,4-dimethylphenyl)-3-methoxy-5-oxo-1,5-dihydropyrazol-4-ylidene]hydrazino}-2-hydroxy-3'-tetrazol-5-ylbiphenyl;
3-{N'-[1-(3,4-dimethylphenyl)-3-ethoxy-5-oxo-1,5-dihydropyrazol-4-ylidene]hydrazino}-2-hydroxy-3'-tetrazol-5-ylbiphenyl;
3-{N'-[1-(3,4-dimethylphenyl)-3-(1-methylethoxy)-5-oxo-1,5-dihydropyrazol-4-ylidene]hydrazino}-2-hydroxy-3'-tetrazol-5-ylbiphenyl;
3-{N'-[1-(4-fluorophenyl)-3-methyl-5-oxo-1,5-dihydropyrazol-4-ylidene]hydrazino}-2-hydroxy-3'-tetrazol-5-ylbiphenyl;
3-{N'-[1-(4-fluoro-3-methylphenyl)-3-methyl-5-oxo-1,5-dihydropyrazol-4-ylidene]hydrazino}-2-hydroxy-3'-tetrazol-5-ylbiphenyl;
3-{N'-[3-methyl-5-oxo-1-(4-trifluoromethylphenyl)-1,5-dihydropyrazol-4-ylidene]hydrazino}-2-hydroxy-3'-tetrazol-5-ylbiphenyl;
3'-{N'-[1-(3.4-dimethylphenyl)-3-(pyridin-4-yl-5-oxo-1,5-dihydropyrazol-4-ylidene]hydrazino}-2'-hydroxybiphenyl-3-carboxylic acid;
3-{N'-[1-(3,4-dimethylphenyl)-3-pyridin-4-yl-5-oxo-1,5-dihydropyrazol-4-ylidene]hydrazino}-2-hydroxy-3'-tetrazol-5-ylbiphenyl;
3-{N'-[1-(3,4-dimethylphenyl)-3-pyridin-2-yl-5-oxo-1,5-dihydropyrazol-4-ylidene]hydrazino}-2-hydroxy-3'-tetrazol-5-ylbiphenyl;
3'-{N'-[1-(3.4-dimethylphenyl)-3-(pyridin-2-yl-5-oxo-1,5-dihydropyrazol-4-ylidene]hydrazino}-2'-hydroxybiphenyl-3-carboxylic acid;
3-{N'-[1-(3-fluoro-4methylphenyl)-3-methyl-5-oxo-1,5-dihydropyrazol-4-ylidene]hydrazino}-2-hydroxy-3'-tetrazol-5-ylbiphenyl;
3'-{N'-[1-(3-fluoro-4-methylphenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(4-trifluoromethylpyrimidin-2-yl)-1,5-dihydropyrazol-4-ylidene]hydrazino}-2'-hydroxybiphenyl-3-carboxylic acid;
3'-N-tert-butoxycarbonylamino-3-{N'-[1-(3,4-Dimethylphenyl)-3-methyl-5-oxo-1,5-dihydropyrazol-4-ylidene]hydrazino}-2-hydroxybiphenyl;
3'-amino-3-{N'-[1-(3,4-dimethylphenyl)-3-methyl-5-oxo-1,5-dihydropyrazol-4-ylidene]hydrazino}-2-hydroxybiphenyl;
3-{N'-[1-(3-fluorophenyl)-3-methyl-5-oxo-1,5-dihydropyrazol-4-ylidene]hydrazino}-2-hydroxy-3'-tetrazol-5-ylbiphenyl;
3'-{N'-[1-(3-fluorophenyl)-3-methyl-5-oxo-1,5-dihydropyrazol-4-ylidene]hydrazino}-2'-hydroxybiphenyl-3-carboxylic acid;
3-{N'-[3-methyl-5-oxo-1-(2,3,4,5,6-pentafluorophenyl)-1,5-dihydropyrazol-4-ylidene]hydrazino}-2-hydroxy-3'-tetrazol-5-ylbiphenyl;
3'-{N'-[3-methyl-5-oxo-1-(2,3,4,5,6-pentafluorophenyl)-1,5-dihydropyrazol-4-ylidene]hydrazino}-2'-hydroxybiphenyl-3-carboxylic acid;
3'-{N'-[1-(3,4-difluorophenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methoxymethyl-5-oxo-1,5-dihydropyrazol-4-ylidene]hydrazino}-2'-hydroxybiphenyl-3-carboxylic acid;
3-{N'-[1-(3,4-dimethylphenyl)-3-methoxymethyl-5-oxo-1,5-dihydropyrazol-4-ylidene]hydrazino}-2-hydroxy-3'-tetrazol-5-ylbiphenyl;
3-{N'-[1-(3,4-difluorophenyl)-3-methyl-5-oxo-1,5-dihydropyrazol-4-ylidene]hydrazino}-2-hydroxy-3'-tetrazol-5-ylbiphenyl;
3'-{N'-[1-(3,4-dimethylphenyl)-5-oxo-3-trifluoromethyl-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-6-fluoro-2'-hydroxybiphenyl-3-carboxylic acid;
3'-{N'-[1-(3,4-dimethylphenyl)-5-oxo-3-propyl-1,5-dihydropyrazol-4-ylidene]hydrazino}-2'-hydroxybiphenyl-3-carboxylic acid;
3-{N'-[1-(3,4-dimethylphenyl)-5-oxo-3-propyl-1,5-dihydropyrazol-4-ylidene]hydrazino}-2-hydroxy-3'-tetrazol-5-ylbiphenyl;
3'-{N'-[1-(3,4-dimethylphenyl)-3-(1-methyl-1H-pyrrol-3-yl)-5-oxo-1,5-dihydropyrazol-4-ylidene]hydrazino}-2'-hydroxybiphenyl-3-carboxylic acid;
3-{N'-[1-(3,4-dimethylphenyl)-3-(1-methyl-1H-pyrrol-3-yl)-5-oxo-1,5-dihydropyrazol-4-ylidene]hydrazino}-2-hydroxy-3'-tetrazol-5-ylbiphenyl;
3'-{N'-[1-(3,4-dimethylphenyl)-3-furan-2-yl-5-oxo-1,5-dihydropyrazol-4-ylidene]hydrazino}-2'-hydroxybiphenyl-3-carboxylic acid;
3-{N'-[1-(3,4-dimethylphenyl)-3-furan-2-yl-5-oxo-1,5-dihydropyrazol-4-ylidene]hydrazino}-2-hydroxy-3'-tetrazol-5-ylbiphenyl;
N-(2'-hydroxy-3'-{N'-[3-methyl-5-oxo-1-(4-trifluoromethyl-phenyl)-1,5-dihydro-pyrazol-4-ylidene]hydrazino}biphenyl-3-yl)-1,1,1-trifluoromethanesulfonamide;
N-(2'-hydroxy-3'-{N'-[1-(3-fluoro-4-methylphenyl)-3-methyl-5-oxo-1,5-dihydro-pyrazol-4-ylidene]hydrazino}biphenyl-3-yl)-1,1,1-trifluoromethanesulfonamide;
N-(2'-hydroxy-3'-{N'-[1-(4-fluoro-3-methylphenyl)-3-methyl-5-oxo-1,5-dihydro-pyrazol-4-ylidene]hydrazino}biphenyl-3-yl)-1,1,1-trifluoromethanesulfonamide;
N-(2'-hydroxy-3'-{N'-[1-(3,4-difluorophenyl)-3-methyl-5-oxo-1,5-dihydro-pyrazol-4-ylidene]hydrazino}biphenyl-3-yl)-1,1,1-trifluoromethanesulfonamide;
N-(3'-{N'-[1-(3,4-dimethylphenyl)-3-methyl-5-oxo-1,5-dihydropyrazol-4-ylidene]hydrazino}-2'-hydroxybiphenyl-3-yl)guanidine;
3'-{N'-[1-(3,4-dimethylphenyl)-3-ethyl-5-oxo-1,5-dihydropyrazol-4-ylidene]hydrazino}-2'-hydroxybiphenyl-3-carboxylic acid;
3-{N'-[1-(3,4-dimethylphenyl)-3-ethyl-5-oxo-1,5-dihydropyrazol-4-ylidene]hydrazino}-2-hydroxy-3'-tetrazol-5-ylbiphenyl;
3'-{N'-[1-(3,4-dimethylphenyl)-5-oxo-3-thien-2-yl-1,5-dihydropyrazol-4-ylidene]hydrazino}-2'-hydroxybiphenyl-3-carboxylic acid;
3'-{N'-[3-cyclopropyl-1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-5-oxo-3-thiazol-2-yl-1,5-dihydropyrazol-4-ylidene]hydrazino}-2'-hydroxybiphenyl-3-carboxylic acid;
3'-{N'-[1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-3-(1-methylethyl)-5-oxo-1,5-dihydropyrazol-4-ylidene]hydrazino}-2'-hydroxybiphenyl-3-carboxylic acid;
3'-{N'-[3-(benzyloxymethyl)-1-(3,4-dimethylphenyl)-5-oxo-1,5-dihydropyrazol-4-ylidene]hydrazino}-2'-hydroxybiphenyl-3-carboxylic acid;
3'-{N'-[3-ethyl-5-oxo-1-(4-trifluoromethylphenyl)-1,5-dihydropyrazol-4-ylidene]hydrazino}-2'-hydroxybiphenyl-3-carboxylic acid;
3'-{N'-[5-oxo-1-(4-trifluoromethylphenyl)-1,5-dihydropyrazol-4-ylidene]hydrazino}-2'-hydroxybiphenyl-3-carboxylic acid;
3'-{N'-[-1-(3,4-dimethylphenyl)-3-hydroxymethyl-5-oxo-1,5-dihydropyrazol-4-ylidene]hydrazino}-2'-hydroxybiphenyl-3-carboxylic acid;
3'-{N'-[3-benzyloxymethyl-5-oxo-1-(4-trifluoromethylphenyl)-1,5-dihydropyrazol-4-ylidene]hydrazino}-2'-hydroxybiphenyl-3-carboxylic acid;
3'-{N'-[-1-(3,4-dimethylphenyl)-3-methylsulfanylmethyl-5-oxo-1,5-dihydropyrazol-4-ylidene]hydrazino}-2'-hydroxybiphenyl-3-carboxylic acid;
3'-{N'-[-1-(3,4-dimethylphenyl)-5-oxo-3-thiophen-3-yl-1,5-dihydropyrazol-4-ylidene]hydrazino}-2'-hydroxybiphenyl-3-carboxylic acid;
3'-{N'-[5-oxo-1-(4-trifluoromethylphenyl)-3-thiophen-3-yl-1,5-dihydropyrazol-4-ylidene]hydrazino}-2'-hydroxybiphenyl-3-carboxylic acid;
3'-{N'-[5-oxo-1-(4-trifluoromethylphenyl)-3-methylsulfanylmethyl-1,5-dihydropyrazol-4-ylidene]hydrazino}-2'-hydroxybiphenyl-3-carboxylic acid;
N-(3'-{N'-[1-(3,4-dimethylphenyl)-3-methyl-5-oxo-1,5-dihydro-pyrazol-4-ylidene]hydrazino}-2'-hydroxybiphenyl-3-yl)methanesulfonamide;
3'-[N'-(1-benzo[1,3]dioxol-5-yl-3-methyl-5-oxo-1,5-dihydropyrazol-4-ylidene)hydrazino]-2'-hydroxybiphenyl-3-carboxylic acid;
3'-{N'-[1-(3,5-dimethylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-4'-hydroxybiphenyl-4-carboxylic acid;
3'-{N'-[1-(3-chloro-4-methylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-4'-hydroxybiphenyl-3-carboxylic acid;
3'-{N'-[1-(3,4-dimethylphenyl)-3-methyl-5-oxo-1,5-dihydropyrazol-4-ylidene]hydrazino}-2'-hydroxybiphenyl-3-phosphonic acid;
3'-{N'-[1-(3,4-dimethylphenyl)-3-methyl-5-oxo-1,5-dihydropyrazol-4-ylidene]hydrazino}-2'-hydroxybiphenyl-3,4-dicarboxylic acid;
2',6-dihydroxy-3'-{N'-[1-(3,4-dimethylphenyl)-3-methyl-5-oxo-1,5-dihydropyrazol-4-ylidene]hydrazino}biphenyl-3-carboxylic acid;
4-aza-3'-{N'-[1-(3,4-dimethylphenyl)-3-methyl-5-oxo-1,5-dihydropyrazol-4-ylidene]hydrazino}-2'-hydroxybiphenyl-5-carboxylic acid;
3'-{N'-[1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-2'-hydroxybiphenyl-3-sulfonic acid; and
5-(3'-{N'-[1-(3,4-Dimethylphenyl)-3-methyl-5-oxo-1,5-dihydropyrazol-4-ylidene]hydrazino}-2'-hydroxybiphenyl-3-ylmethylene)thiazolidine-2,4-dione; and/or pharmaceutically acceptable salts, hydrates, solvates and esters thereof.

Included among the non-peptide TPO receptor agonists of the invention are the non-peptide compounds described in:
WO 02/59099;
WO 02/59100;
EP 1 207 155;
EP 1 253 142A1;
WO 01/92211 A1;
WO 01/53267-A1;
EP 1 104 674- A1; and
WO 01/07423-A1.

Included among the compounds of the above listed applications that are useful in the present invention are:
N-[4-(5-bromo-2-thienyl)-1,3-thiazol-2-yl]-4-[(Z)-(2,4-dioxo-1,3-thiazolidin-5-ylidene)methyl]benzamide;
N-[4-(3,4-dimethylphenyl)-1,3-thiazol-2-yl]-4-[(Z)-(2,4-dioxo-1,3-thiazolidin-5-ylidene)methyl]benzamide;
N-{4-[4-(1,1-dimethylethyl)phenyl]-1,3-thiazol-2-yl}-4-[(Z)-(2,4-dioxo-1,3-thiazolidin-5-ylidene)methyl]benzamide;
N-[4-(3,4-dichlorophenyl)-1,3-thiazol-2-yl]-4-[(Z)-(2,4-dioxo-1,3-thiazolidin-5-ylidene)methyl]benzamide; and
(2E)-3-[4-({[4-(3,4-dichlorophenyl)-1,3-thiazol-2-yl]amino}carbonyl)phenyl]-2-methyl-2-propenoic acid;
   and/or pharmaceutically acceptable salts, hydrates, solvates and esters thereof.

Included among the non-peptide TPO receptor agonists of the invention are the non-peptide compounds described in:
WO 99/11262.

Non-peptide TPO receptor agonists are included in the pharmaceutical compositions of the invention and used in the methods of the invention.

By the term "protected hydroxy" or "protected -OH" as used herein, is meant the alcoholic or carboxylic-OH groups which can be protected by conventional blocking groups in the art such as described in "Protective Groups In Organic Synthesis" by Theodora W. Greene, Wiley-Interscience, 1981, New York. Compounds containing protected hydroxy groups may also be useful as intermediates in the preparation of the pharmaceutically active compounds of the invention.

By the term "aryl" as used herein, unless otherwise defined, is meant a cyclic or polycyclic aromatic ring containing from 1 to 14 carbon atoms and optionally containing from one to five heteroatoms, provided that when the number of carbon atoms is 1 the aromatic ring contains at least four heteroatoms, when the number of carbon atoms is 2 the aromatic ring contains at least three heteroatoms, when the number of carbons is 3 the aromatic ring contains at least two heteroatoms and when the number of carbon atoms is 4 the aromatic ring contains at least one heteroatom.

By the term "C₁-C₁₂aryl" as used herein, unless otherwise defined, is meant phenyl, naphthalene, 3,4-methylenedioxyphenyl, pyridine, biphenyl, quinoline, pyrimidine, quinazoline, thiophene, furan, pyrrole, pyrazole, imidazole and tetrazole.

When referring to compounds of Formula (I) and (II), the term "substituted" as used herein, unless otherwise defined, is meant that the subject chemical moiety has one or more substituents selected from the group consisting of: -CO₂R²⁰, aryl, -C(O)NHS(O)₂R²⁰, -NHS(O)₂R²⁰, hydroxyalkyl, alkoxy, -C(O)NR²¹ R²², acyloxy, alkyl, amino, N-acylamino, hydroxy, -(CH₂)_{g}C(O)OR⁸, -S(O)ₙR⁸, nitro, tetrazole, cyano, oxo, halogen, trifluoromethyl, protected -OH and a heterocyclic methylene substituent as represented by Formula (III), 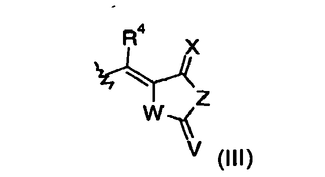 , where g is 0-6; R⁸ is hydrogen or alkyl; R²⁰ is selected form hydrogen, C₁-C₄alkyl, aryl and trifluoromethyl; R²¹ and R²² are independently selected form hydrogen, C₁-C₄alkyl, aryl and trifluoromethyl; V, W, X and Z are each independently selected from O, S, and NR¹⁶, where R¹⁶ is selected from: hydrogen, alkyl, cycloalkyl, C₁-C₁₂aryl, substituted alkyl, substituted cycloalkyl and substituted C₁-C₁₂aryl; and n is 0-2.

When referring to compounds of Formula (V) and (VI), the term "substituted" as used herein, unless otherwise defined, is meant that the subject chemical moiety has one or more substituents selected from the group consisting of: -CO₂R²⁰, aryl, -C(O)NHS(O)₂R²⁰, -NHS(O)₂R²⁰, hydroxyalkyl, alkoxy, - C(O)NR²¹ R²², acyloxy, alkyl, amino, N-acylamino, hydroxy, -(CH₂)_{g}C(O)OR⁸, -S(O)ₙR⁸, nitro, tetrazole, cyano, oxo, halogen, trifluoromethyl and protected -OH, where g is 0-6, R⁸ is hydrogen or alkyl, R²⁰ is selected form hydrogen, C₁-C₄alkyl, aryl and trifluoromethyl, and R²¹ and R²² are independently selected form hydrogen, C₁-C₄alkyl, aryl and trifluoromethyl, and n is 0-2.

By the term "alkoxy" as used herein is meant -Oalkyl where alkyl is as described herein including -OCH₃ and -OC(CH₃)₂CH₃.

The term "cycloalkyl" as used herein unless otherwise defined, is meant a nonaromatic, unsaturated or saturated, cyclic or polycyclic C₃-C₁₂.

Examples of cycloalkyl and substituted cycloalkyl substituents as used herein include: cyclohexyl, 4-hydroxy-cyclohexyl, 2-ethylcyclohexyl, propyl 4-methoxycyclohexyl, 4-methoxycyclohexyl, 4-carboxycyclohexyl, cyclopropyl and cyclopentyl.

By the term "acyloxy" as used herein is meant -OC(O)alkyl where alkyl is as described herein. Examples of acyloxy substituents as used herein include: - OC(O)CH₃, -OC(O)CH(CH₃)₂ and -OC(O)(CH₂)₃CH₃.

By the term "N-acylamino" as used herein is meant -N(H)C(O)alkyl, where alkyl is as described herein. Examples of N-acylamino substituents as used herein include: -N(H)C(O)CH₃, -N(H)C(O)CH(CH₃)₂ and -N(H)C(O)(CH₂)₃CH₃.

By the term "aryloxy" as used herein is meant -Oaryl where aryl is phenyl, naphthyl, 3,4-methylenedioxyphenyl, pyridyl or biphenyl optionally substituted with one or more substituents selected from the group consisting of: alkyl, hydroxyalkyl, alkoxy, trifuloromethyl, acyloxy, amino, N-acylamino, hydroxy, -(CH₂)_{g}C(O)OR⁸, - S(O)ₙR⁸, nitro, cyano, halogen and protected -OH, where g is 0-6, R⁸ is hydrogen or alkyl, and n is 0-2. Examples of aryloxy substituents as used herein include: phenoxy, 4-fluorophenyloxy and biphenyloxy.

By the term "heteroatom" as used herein is meant oxygen, nitrogen or sulfur.

By the term "halogen" as used herein is meant a substituent selected from bromide, iodide, chloride and fluoride.

By the term "alkyl" and derivatives thereof and in all carbon chains as used herein is meant a linear or branched, saturated or unsaturated hydrocarbon chain, and unless otherwise defined, the carbon chain will contain from 1 to 12 carbon atoms. Examples of alkyl substituents as used herein include: -CH₃, -CH₂-CH₃, -CH₂-CH₂-CH₃, -CH(CH₃)₂, -C(CH₃)₃, -(CH₂)₃-CH₃, -CH₂-CH(CH₃)₂. -_{CH}(_{CH3})-_{C}H₂-CH₃, -CH=CH₂, and -C≡C-CH₃.

By the term "treating" and derivatives thereof as used herein, is meant prophylatic and therapeutic therapy.

By the phrases "to a therapeutic extent" and "therapeutically effective amount" and derivatives thereof as used herein, unless otherwise defined, is meant that the incidence of degenerative disease/injury in patients treated with a non-peptide TPO receptor agonist is prevented or reduced in severity in comparison to untreated patients.

By the phrase "non-peptide" as used herein is meant a chemical compound, or a protein or peptide not comprised primarily of natural amino acids. Suitably, the "non-peptide" is a small molecule chemical compound having a molecular weight under 1,500 daltons, suitably under 1,000 daltons.

By the term "primarily" as used above is meant about 60% by weight of naturally occurring amino acid residue.

By the phrase "degenerative diseases/injuries" and derivatives thereof as used herein, unless otherwise defined, is meant: nervous system disorders, including transverse myelitis, multiple sclerosis, demyelination occurring after trauma to the brain or spinal cord, acute brain injury, head trauma, spinal cord injury, peripheral nerve injury, ischaemic brain injury, hereditary myelin disorder of the CNS, epilepsy, perinatal asphxia, asphyxia, anoxia, status epilepticus, and stroke; baldness, such as male pattern baldness and alopecia areata; neurodegenerative diseases, such as Alzheimer's disease, Parkinson disease, Huntington's disease, and amyotrophic lateral sclerosis; tissue reparation disorders, including cardiovascular disorders, myocardial infarction, cardiovascular disease, gastrointestinal disease, kidney disease and liver disease; damaged tissue, such as flesh wounds, age damaged cells and age damaged tissue; lupus; and diabetes/diabetes mellitus.

As used herein stroke refers to a Cerebral Vascular Incident and includes acute thromboembolic stroke. The term stroke, as used herein, also includes both focal and global ischemia. Also included in stroke, as used herein, are transient cerebral ischemic attacks and other cerebral vascular problems accompanied by cerebral ischemia. A patient undergoing carotid endarterectomy specifically or other cerebrovascular or vascular surgical procedures in general, or diagnostic vascular procedures including cerebral angiography and the like, are also examples of stroke, as used herein.

Injuries that are included within the term "degenerative diseases/injuries" are: head trauma, spinal cord trauma and injury from general anoxia, hypoxia, hypoglycemia, hypotension, as well as similar injuries seen during procedures from embole, hyperfusion, and hypoxia.

Further injuries treatable by the present invention include those which occur, during cardiac bypass surgery, in incidents of intracranial hemorrhage, in perinatal asphyxia, in cardiac arrest, and status epilepticus.

Additional degenerative diseases treatable by the present invention are disease states caused by excessive bone loss or cartilage or matrix degradation such as: osteoporosis, glucocorticoid induced osteoporosis, Paget's disease, abnormally increased bone turnover, periodontal disease, gingivitis, tooth loss, bone fractures, arthritis, rheumatoid arthritis, osteoarthritis, periprosthetic osteolysis, osteogenesis imperfecta, or metastatic bone disease. It is part of the present invention that treatment with a non-peptide TPO receptor agonist, as described herein, is useful in reducing the risk of bone fractures and in increasing bone mineral density.

Additional degenerative diseases treatable by the present invention are degenerative diseases of the eye such as: macular degeneration, dry eye syndrome, cataracts, diabetic retinopathy, glaucoma, vitreous disease and retinal degeneration.

An additional degenerative disease treatable by the present invention is AIDS.

Because the in vivo administration of the non-peptide TPO receptor agonist of the present invention, in mammals, including humans, exhibits therapeutic activity in diseases/injuries that are therapeutically treatable by stem cells/stem cell therapy, the non-peptide TPO receptor agonist of the present invention are useful in treating diseases/injuries that are known to be treatable by stem cells/stem cell therapy or found to be treatable by stem cells/stem cell therapy.

An example of damaged tissue, such as flesh wounds, as used herein is vascular access dysfunction in mammals, including humans. Suitably, the vascular access dysfunction is in association with the insertion, maintenance or repair of an indwelling shunt, fistula or catheter, suitably a large bore catheter, into a vein.

Further, vascular access dysfunction in chemotherapy patients is generally caused by outflow stenoses in the venous circulation and results in a decreased ability to administer medications to cancer patients. Often the outflow stenoses is so severe as to require intervention.

Additionally, vascular access dysfunction in total parenteral nutrition (TPN) patients is generally caused by outflow stenoses in the venous circulation and results in reduced ability to care for these patients.

The current invention is directed to the prevention or reduction of vascular access dysfunction in association with the insertion or repair of an indwelling shunt, fistula or catheter, suitably a large bore catheter, into a vein in a mammal, particularly a human patient.

By the phrase "prevention or reduction of vascular access dysfunction in association with the insertion or repair of an indwelling shunt, fistula or catheter" as used herein, is meant that the incidence of vascular thrombosis and/or fistula failure and/or shunt failure and/or vascular access clotting and/or stenosis and/or restenosis and/or the need for declotting an indwelling vascular access shunt, fistula or catheter in patients treated with a non-peptide TPO receptor agonist collected over the observation period are prevented or reduced in comparison to untreated patients.

By the term "collected over the observation period" as used herein, means a period of up to or about 12 months, preferably 12 months.

An example of damaged tissue, such as flesh wounds, as used herein is restenosis associated with arterial coronary intervention, suitably the insertion of a stent. The current invention is directed to the inhibition of restenosis associated with arterial coronary intervention.

An example of damaged tissue, such as flesh wounds, as used herein is peripheral vascular disease in mammals, including humans. By the term "peripheral vascular disease" and derivatives thereof, as used herein, is meant a non-coronary artery that has undergone percutaneous intervention, with or without stent placement, suitably, the intervention was due to a disease state selected form: renal artery stenosis; in cerebral vessels - carotid artery stenosis and vertebral arteries; and peripheral atherosclerosis in vessels, preferably the internal iliac artery, the femoral artery or in mesenteric vessels. Treatment of peripheral vascular disease with a non-peptide TPO receptor agonist will be similar to the treatment of vascular access dysfunction as described above.

When describing the treatment of damaged tissue, such as flesh wounds, with a non-peptide TPO receptor agonist, as described herein, a favorable result is a decrease in scaring.

When describing treatment, particularly of age damaged tissue, with a non-peptide TPO receptor agonist, as described herein, a favorable result is prolonging the life of the subject.

When describing treatment, particularly of Alzheimer's disease, with a non-peptide TPO receptor agonist, as described herein, a favorable result is the enhancement of memory and/or cognitive function of the subject.

All publications, including but not limited to patents and patent applications, cited in this specification are herein incorporated by reference as though fully set forth.

Compounds of Formula (I) are included in the pharmaceutical compositions of the invention and used in the methods of the invention. Where a -COOH or -OH group is present, pharmaceutically acceptable esters can be employed, for example methyl, ethyl, pivaloyloxymethyl, and the like for -COOH, and acetate maleate and the like for -OH, and those esters known in the art for modifying solubility or hydrolysis characteristics, for use as sustained release or prodrug formulations.

The compounds of Formulas I and II are disclosed and claimed, along with pharmaceutically acceptable salts, hydrates, solvates and esters thereof, as being useful as an agonist of the TPO receptor, particularly in enhancing platelet production and particularly in the treatment of thrombocytopenia, in International Application No. PCT/US01/16863, having an International filing date of May 24, 2001; International Publication Number WO 01/89457 and an International Publication date of November 29, 2001, the entire disclosure of which is hereby incorporated by reference. Compounds of Formulas I and II and pharmaceutically acceptable salts, hydrates, solvates and esters thereof, are prepared as described in International Application No. PCT/US01/16863. The bis-(monoethanolamine) salt of a compound described in International Application No. PCT/US01/16863, is described in International Application No. PCT/US03/16255, having an International filing date of May 21, 2003; International Publication Number WO 03/098992 and an International Publication date of December 4, 2003.

The treatment of degenerative diseases/injuries, as described herein, is accomplished by the administration of a non-peptide TPO receptor agonist and is not limited to any particular mechanism of action. A mechanism of action for treating degenerative diseases/injuries, as described herein, is by stimulating the survival and/or production of stem cells and/or increasing stem cell function and/or longevity to a therapeutic extent.

By the term "co-administering" and derivatives thereof as used herein is meant either simultaneous administration or any manner of separate sequential administration of a TPO receptor agonist, as described herein, and a further active ingredient or ingredients, known to treat degenerative diseases/injuries. Preferably, if the administration is not simultaneous, the compounds are administered in a close time proximity to each other. Furthermore, it does not matter if the compounds are administered in the same dosage form, e.g. one compound may be administered topically and another compound may be administered orally.

Examples of a further active ingredient or ingredients for use in combination with non-peptide TPO receptor agonists according to the present invention include but are not limited to: chemoprotective or myeloprotective agents such as G-CSF, BB10010 (Clemons et al., Breast Cancer Res. Treatment, 1999, 57, 127), amifostine (Ethyol) (Fetscher et al., Current Opinion in Hemat., 2000, 7, 255-60), SCF, IL-11, MCP-4, IL-1-beta, AcSDKP (Gaudron et al., Stem Cells, 1999, 17, 100-6), TNF-a, TGF-b, MIP-1a (Egger et al., Bone Marrow Transpl., 1998, 22 (Suppl. 2), 34-35), and other molecules identified as having anti-apoptotic, survival or proliferative properties.

Tpo has been demonstrated to act as a mobilizer of stem cells into the peripheral blood (Neumann T. A. et al., Cytokines, Cell. & Mol. Ther., 2000, 6, 47-56). This activity can synergize with stem cell mobilizers such as G-CSF (Somolo et al., Blood, 1999, 93, 2798-2806). The TPO receptor agonists of the present invention are useful in increasing the numbers of stem cells in circulation in donors prior to leukapheresis for hematopoietic stem-cell transplantation in patients receiving myelo-ablative chemotherapy.

Likewise, TPO stimulates growth of myeloid cells, particularly those of granulocyte/macrophage lineage (Holly et al., US-5989537). Granulocyte/macrophage progenitors are cells of the myeloid lineage that mature as neutrophils, monocytes, basophils and eosinophils. The compounds described in the present invention have therapeutic utility in stimulating the poliferation of neutrophils in patients with neutropenic conditions.

Further, compounds that treat diseases caused by excessive bone loss or cartilage or matrix degradation are known to be used in combination with further active ingredients. (PCT/US03/06147, having an International filing date of February 28, 2003). According to the present invention, non-peptide TPO receptor agonists are useful when administered with further active compounds known to treat diseases caused by excessive bone loss or cartilage or matrix degradation, such as: an organic bisphosphonate, an estrogen receptor modulator, an androgen receptor modulator, an inhibitor of osteoclast proton ATPase, an inhibitor of HMG-CoA reductase, an integrin receptor antagonist, or an osteobalst anabolic agent.

It is part of this discovery that the in vivo administration of non-peptide TPO receptor agonists is useful in treating Parkinson's disease, Huntingtion's disease, multiple sclerosis and ischaemic brain injury. Stem cells, including adult bone marrow stem cells are indicated as effective in treating multiple sclerosis; Stangel M. et al., Progress in Neurobiology, 68(5): 361-76, 2002 Dec. Neural stem cells and their use in Parkinson's disease, Huntingtion's disease, multiple sclerosis and ischaemic brain injury is described in Ostenfield T. et al., Advances & Technical standards in Neurosuraery, 28: 3-89, 2003.

Further, it is part of this discovery that the in vivo administration of non-peptide TPO receptor agonists are useful in the regeneration and repair of tissues that respond to stem cell treatment. Such tissues are readily known or readily ascertainable by those skilled in the art. For example, stem cells are indicated as being useful in treating patients with myocardial infarction, cardiovascular disorders and cardiovascular disease; Stamm C. et al., Lancet. 361 (9351): 45-6, 2003 and Semsarian C., Internal Medicine Journal. 32(5-6): 259-65, 2002. Stem cells are indicated in treating, repairing and/or in the regeneration of liver disease/tissue, gastrointestinal disease/tissue and kidney disease/tissue; Choi D. et al., Cell transplantation, 11(4): 359-68, 2002, Poulsom R. et al., Journal of Pathology, 197 (4): 441-56, 2002 and Alison M. et al., Journal of Pathology, 197 (4): 419-23, 2002.

Further, it is part of this discovery that the in vivo administration of non-peptide TPO receptor agonists are useful in the treatment of diabetes/diabetes mellitus. Stem cells are indicated in treating diabetes, Berna G, et al., Biomedicine & Pharmacotherapy, 55(4): 206-12, 2001 and Beilhack GF., et al., Diabetes, 52(1):59-68, 2003.

Additional examples of a further active ingredient or ingredients for use in combination with non-peptide TPO receptor agonists according to the present invention include but are not limited to: stem cell, megakaryocyte, neutrophil mobilizers such as chemotherapeutic agents (i.e., cytoxan, etoposide, cisplatin, Ballestrero A. et al., Oncology, 2000, 59, 7-13), chemokines, IL-8, Gro-beta (King, A. G. et al. J. lmmun., 2000, 164, 3774-82), receptor agonist or antagonist antibodies, small molecule cytokine or receptor agonists or antagonists, SCF, Fit3 ligand, adhesion molecule inhibitors or antibodies such as: anti-VLA-4 (Kikuta T. et al., Exp. Hemat., 2000, 28, 311-7) or anti-CD44 (Vermeulen M. et al., Blood, 1998, 92, 894-900), cytokine/chemokine/interleukin or receptor agonist or antagonist antibodies, MCP-4 (Berkhout TA., et al., J. Biol. Chem., 1997, 272, 16404-16413; Uguccioni M. et al., J. Exp. Med., 1996, 183, 2379-2384).

Because the pharmaceutically active compounds of the present invention are active as TPO receptor agonists they exhibit therapeutic utility in treating degenerative diseases/injuries.

Degenerative diseases are known to have many causative factors, including but not limited to, viral infections (including, but not limited to; HIV, hepatitis C, parvovirus) and liver disease, aging, auto immune diseases, neural disease/damage, liver disease/damage, kidney disease/damage, gastrointestinal disease/damage, cardiovascular disease/damage and pancreatic disease/damage. This invention relates to the treatment of degenerative diseases regardless of the factor or factors causing the condition. The pharmaceutically active compounds of this invention are also useful in treating degenerative diseases when the causative factor or factors of the condition are unknown or have yet to be identified.

A skilled physician will be able to determine the appropriate situation in which subjects are susceptible to or at risk of, for example, stroke as well as suffering from stroke for administration by methods of the present invention.

Prophylactic use of the compounds of this invention is contemplated whenever a degenerative disease/injury is anticipated. Prophylactic uses of the compounds of this invention includes but is not limited to transplant surgery, surgery, anesthesia prior to child birth and gut protection.

TPO is known to have various effects including anti-apototic/survivat effects on megakaryocytes, platelets and stem cells, and proliferative effects on stem cells and megakaryocytic cells (Kuter D. J. Seminars in Hematology, 2000, 37, 41-9). These TPO activities effectively increase the number of stem and progenitor cells so that there is synergistic effects when TPO is used in conjunction with other cytokines that induce differentiation.

The non-peptide TPO receptor agonists of the current invention are also useful in acting on cells for survival and/or proliferation in conjunction with other agents known to act on cells for survival and/or proliferation. Such other agents include but are not limited to: G-CSF, GM-CSF, TPO, M-CSF, EPO, Gro-beta, IL-11, SCF, FLT3 ligand, LIF, IL-3, IL-6, IL-1, Progenipoietin, NESP, SD-01, or IL-5 or a biologically active derivative of any of the aforementioned agents, KT6352 (Shiotsu Y. et al., Exp. Hemat. 1998, 26, 1195-1201), uteroferrin (Laurenz JC., et al. Comp. Biochem. & Phys., Part A. Physiology., 1997, 116, 369-77), FK23 (Hasegawa T., et al. Int. J. Immunopharm., 1996, 18 103-112) and other molecules identified as having anti-apoptotic, survival or proliferative properties for stem cells, progenitor cells, or other cells expressing TPO Receptors.

The non-peptide TPO receptor agonist of this invention interact differently at the TPO receptor than does TPO. One result of this differing interaction is that the non-peptide TPO receptor agonist of this invention are useful in combination with TPO.

One skilled in the art can readily determine by known methods if a compound is a non-peptide TPO receptor agonist and thus included within the scope of the current invention. By way of example, the following assays can be employed:

### Luciferase Assay

Compounds are tested for potency as agonists of the TPO receptor in a Luciferase assay such as described in Lamb, et al., Nucleic Acids Research 23: 3283-3289 (1995) and Seidel, et al., Proc. Natl. Acad. Sci., USA 92: 3041-3045 (1995) by substituting a TPO-responsive BaF3 cell line (Vigon et al. Proc. Natl. Acad. Sci. USA 1992, 89, 5640-5644) for the HepG2 cells utilized therein. The murine BaF3 cells express TPO receptors and closely match the pattern of STAT (signal transducers and activators of transcription) activation observed in primary murine and human bone marrow cells.

### Proliferation Assay

Compounds are tested in an in vitro proliferation assay using the human UT7TPO cell line. UT7TPO cells are a human megakaryoblastic cell line that express Tpo-R, whose survival and growth is dependent on the presence of TPO (Komatsu et al. Blood 1996, 87,4552).

### Differentiation Assay

Compounds are tested for their ability in stimulating the maturation of megakaryocytes from human bone marrow cells. In this assay, purified human CD34+ progenitor cells are incubated in liquid culture with test compounds for 10 days and the number of cells expressing the transmembrane glycoprotein CD41 (gpllb), a megakaryocytic marker, is then measured by flow cytometry (see Cwirla, S. E. et al Science, 1997, 276, 1696).

The pharmaceutically active compounds within the scope of this invention are useful as non-peptide TPO receptor agonists in mammals, particularly humans, in need thereof.

The ability of non-peptide TPO receptor agonists to treat degenerative diseases/injuries is demonstrated by activity in the CD34+ Progenitor Cell Proliferation Assay.

### CD34+ Progenitor Cell Proliferation Assay

Compounds are tested for their ability in stimulating the survival and proliferation of early CD34+ progenitor cells from human bone marrow. In this assay, purified human CD34+ progenitor cells are incubated in liquid culture with test compounds for up to 7 days and the number of cells expressing the early stem cell marker CD34 are then measured by flow cytometry and compared to untreated cells (see Liu et al. Bone Marrow Transplantation. 24:247-52, 1999). The compound 3'-{N'-[3-methyl-5-oxo-1-(4-trifluoromethylphenyl)-1,5-dihydropyrazol-4-ylidene]hydrazinol-2'-hydroxybiphanyl-3-carboxylic acid was tested in the CD34+ Progenitor Cell Proliferation Assay and at 3 uM increased the number of CD34+ cells in liquid culture by up to 2-fold over vehicle controls at days 2, 5 and 7. rhTpo (100 ng/ml) also demonstrated a 2-fold increase in the number of CD34+ cells in this experiment.

Some of the compounds within the scope of the invention were tested and showed activation from about 4% to 100% of control at a concentration of 0.001-10 uM in the luciferase assay. Some of the compounds of the invention also promoted the proliferation of 32D-mpl cells at a concentration of 0.003 to 30 uM. Some of the compounds of the invention also showed activity in the CD41 megakaryocytic assay at a concentration of 0.003 to 30 uM.

The present invention therefore provides a method of treating a disease/injury state selected from: nervous system disorders, including transverse myelitis, multiple sclerosis, demyelination occurring after trauma to the brain or spinal cord, acute brain injury, head trauma, spinal cord injury, peripheral nerve injury, ischaemic brain injury, hereditary myelin disorder of the CNS, epilepsy, perinatal asphxia, asphyxia, anoxia, status epilepticus, and stroke; baldness, such as male pattern baldness and alopecia areata; neurodegenerative diseases, such as Alzheimer's disease, Parkinson disease, Huntington's disease, and amyotrophic lateral sclerosis; in the treatment, repair and/or regeneration of tissue, for example: in cardiovascular disorders, myocardial infarction and cardiovascular disease/tissue (hereinafter cardiovascular disease), and in the treatment, repair and/or regeneration of liver disease/tissue (hereinafter liver disease), gastrointestinal disease/tissue (hereinafter gastrointestinal disease) and kidney disease/tissue (hereinafter kidney disease); in the restoration of damaged tissue, such as healing flesh wounds, regenerating age damaged cells and regenerating age damaged tissue; in the treatment of lupus; and in the treatment of diabetes/diabetes mellitus which comprises the administration an effective amount of a non-peptide TPO receptor agonist.

The present invention also provides a method of treating degenerative diseases caused by excessive bone loss or cartilage or matrix degradation, such as: osteoporosis, glucocorticoid induced osteoporosis, Paget's disease, abnormally increased bone turnover, periodontal disease, gingivitis, tooth loss, bone fractures, arthritis, rheumatoid arthritis, osteoarthritis, periprosthetic osteolysis, osteogenesis imperfect, or metastatic bone disease, which comprises the administration of an effective amount of a non-peptide TPO receptor agonist.

The present invention also provides a method of treating degenerative diseases of the eye such as: macular degeneration, dry eye syndrome, cataracts, diabetic retinopathy, glaucoma, vitreous disease and retinal degeneration, which comprises the administration of an effective amount of a non-peptide TPO receptor agonist.

The present invention also provides a method of treating AIDS, which comprises the administration of an effective amount of a non-peptide TPO receptor agonist.

The present invention therefore provides a method of treating degenerative diseases/injuries, which comprises the administration of a therapeutically effective amount of a non-peptide TPO receptor agonist, suitably a compound of Formula (I), and/or a pharmaceutically acceptable salt, hydrate, solvate or ester thereof. The drug may be administered to a patient in need thereof by any conventional route of administration, including, but not limited to, intravenous, intramuscular, oral, subcutaneous, intradermal, and parenteral.

The non-peptide TPO receptor agonists of the present invention are incorporated into convenient dosage forms such as capsules, tablets, or injectable preparations. Solid or liquid pharmaceutical carriers are employed. Solid carriers include, starch, lactose, calcium sulfate dihydrate, terra alba, sucrose, talc, gelatin, agar, pectin, acacia, magnesium stearate, and stearic acid. Liquid carriers include syrup, peanut oil, olive oil, saline, and water. Similarly, the carrier or diluent may include any prolonged release material, such as glyceryl monostearate or glyceryl distearate, alone or with a wax. The amount of solid carrier varies widely but, preferably, will be from about 25 mg to about 1 g per dosage unit. When a liquid carrier is used, the preparation will be in the form of a syrup, elixir, emulsion, soft gelatin capsule, sterile injectable liquid such as an ampoule, or an aqueous or nonaqueous liquid suspension.

The pharmaceutical preparations are made following conventional techniques of a pharmaceutical chemist involving mixing, granulating, and compressing, when necessary, for tablet forms, or mixing, filling and dissolving the ingredients, as appropriate, to give the desired oral or parenteral products.

Doses of the pharmaceutically active compounds in a pharmaceutical dosage unit as described above will be an efficacious, nontoxic quantity preferably selected from the range of 0.001 - 100 mg/kg of active compound, preferably 0.002 - 50 mg/kg. When treating a human patient in need of a non-peptide TPO receptor agonist, the selected dose is administered preferably from 1-6 times daily, orally or parenterally. Preferred forms of parenteral administration include topically, rectally, transdermally, by injection and continuously by infusion. Oral dosage units for human administration preferably contain from 0.05 to 3500 mg, more preferably 0.1 to 3000 mg of active compound. Oral administration, which uses lower dosages is preferred. Parenteral administration, at high dosages, however, also can be used when safe and convenient for the patient.

Optimal dosages to be administered may be readily determined by those skilled in the art, and will vary with the particular non-peptide TPO receptor agonist in use, the strength of the preparation, the mode of administration, and the advancement of the disease condition. Additional factors depending on the particular patient being treated will result in a need to adjust dosages, including patient age, weight, diet, and time of administration.

The method of this invention of treating degenerative diseases/injuries in mammals, including humans, comprises administering to a subject in need thereof a therapeutically effective amount of a pharmaceutically active compound of the present invention.

The invention also provides for the use of a compound of Formula (I) in the manufacture of a medicament for use in the treatmet of degenerative diseases/injuries.

The invention also provides for the use of a compound of Formula (I) in the manufacture of a medicament for use in therapy.

The invention also provides for a pharmaceutical composition for use in the treatment of degenerative diseases/injuries which comprises a compound of Formula (I) and a pharmaceutically acceptable carrier.

The invention also provides for the use of a compound of Formula (II) in the manufacture of a medicament for use in the treatmet of degenerative diseases/injuries.

The invention also provides for the use of a compound of Formula (II) in the manufacture of a medicament for use in therapy.

The invention also provides for a pharmaceutical composition for use in the treatment of degenerative diseases/injuries which comprises a compound of Formula (II) and a pharmaceutically acceptable carrier.

No unacceptable toxicological effects are expected when compounds of the invention are administered in accordance with the present invention.

In addition, the pharmaceutically active compounds of the present invention can be co-administered with further active ingredients, such as other compounds known to treat degenerative diseases/injuries or compounds known to have utility when used in combination with a non-peptide TPO receptor agonist.

Contemplated Equivalents - It will be appreciated by the person of ordinary skill in the art that the compounds of Formulas I and II may also exist in tautomeric forms. For example, in Formula I, the double bond that is drawn between the two nitrogen atoms exists between the lower nitrogen atom and the AR substituent. Tautomeric forms of the compounds of Formulas I and II are exemplified by the following Formula (IV): 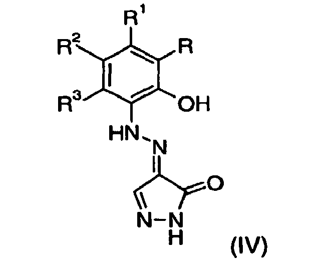 where the 'R' groups are as defined above. All such compounds are included in the scope of the invention and inherently included in the definition of the compounds of Formulas I and II.

Without further elaboration, it is believed that one skilled in the art can, using the preceding description, utilize the present invention to its fullest extent. The following Examples are, therefore, to be construed as merely illustrative and not a limitation of the scope of the present invention in any way.

### Experimental Details

### Example 1- Capsule Composition

An oral dosage form for administering the present invention is produced by filing a standard two piece hard gelatin capsule with the ingredients in the proportions shown in Table I, below.

**Table I**

| INGREDIENTS | AMOUNTS |
|---|---|
| 4'-{N'-[1-(3,4-Dimethylphenyl)-3-methyl-5-oxo-1,5- dihydropyrazol-4-ylidene]hydrazino}-3'-hydroxybiphenyl-4- | 25 mg |
| carboxylic acid | |
| Lactose | 55 mg |
| Talc | 16 mg |
| Magnesium Stearate | 4 mg |

### Example 2 - Injectable Parenteral Composition

An injectable form for administering the present invention is produced by stirring 1.5% by weight of 4'-{N'-[1-(3,4-dimethylpheny)-3-methyl-5-oxo-1,5-dihydropyrazol-4-ylidene]hydrazino}-3'-hydroxybiphenyl-3-carboxylic acid in 10% by volume propylene glycol in water.

### Example 3 - Tablet Composition

The sucrose, calcium sulfate dihydrate and a non-peptide TPO agonist, as shown in Table II below, are mixed and granulated in the proportions shown with a 10% gelatin solution. The wet granules are screened, dried, mixed with the starch, talc and stearic acid, then screened and compressed into a tablet.

**Table II**

| INGREDIENTS | AMOUNTS |
|---|---|
| 3'-{N'-[1-(3,4-dimethylphenyl)-3-methyl-5-oxo-1,5- dihydropyrazol-4-ylidene]hydrazino}-2'-hydroxybiphenyl-3- | 20 mg |
| carboxylic acid | |
| calcium sulfate dihydrate | 30 mg |
| sucrose | 4 mg |
| starch | 2 mg |
| talc | 1 mg |
| stearic acid | 0.5 mg |

While the preferred embodiments of the invention are illustrated by the above, it is to be understood that the invention is not limited to the precise instructions herein disclosed and that the right to all modifications coming within the scope of the following claims is reserved.

## Claims

1. Use of TPO receptor agonist having the following Formula (I): 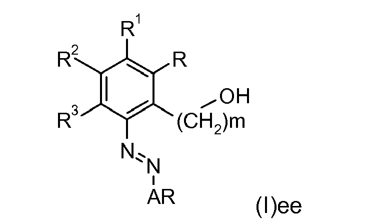 wherein:
R, R¹, R² and R³ are each independently selected from hydrogen, C₁₋₆alkyl, -(CH₂)ₚOR⁴, -C(O)OR⁴, formyl, nitro, cyano, halogen, aryl, substituted aryl, substituted alkyl, -S(O)ₙR⁴, cycloalkyl, -NR⁵R⁶, protected -OH, -CONR⁵R⁶, phosphonic acid, sulfonic acid, phosphinic acid, -SO₂NR⁵R⁶, and a heterocyclic methylene substituent as represented by Formula (III), 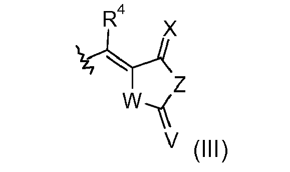 where,
p is 0-6,
n is 0-2,
V, W, X and Z are each independently selected from O, S and NR¹⁶, where R¹⁶ is selected from: hydrogen, alkyl, cycloalkyl, C₁-C₁₂aryl, substituted alkyl, substituted cycloalkyl and substituted C₁-C₁₂aryl,
R⁴ is selected from: hydrogen, alkyl, cycloalkyl, C₁-C₁₂aryl, substituted alkyl, substituted cycloalkyl and substituted C₁-C₁₂aryl, and
R⁵ and R⁶ are each independently selected from hydrogen, alkyl, substituted alkyl, C₃-₆cycloalkyl, and aryl,
or R⁵ and R⁶ taken together with the nitrogen to which they are attached represent a 5 to 6 member saturated ring containing up to one other heteroatom selected from oxygen and nitrogen;
m is 0-6; and
AR is a cyclic or polycyclic aromatic ring containing from 3 to 16 carbon atoms and optionally containing one or more heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, substituted alkyl, aryl, substituted cycloalkyl, substituted aryl, aryloxy, oxo, hydroxy, alkoxy, cycloalkyl, acyloxy, amino, N-acylamino, nitro, cyano, halogen, -C(O)OR⁴, -C(O)NR¹⁰R¹¹, -S(O)₂NR¹⁰R¹¹, - S(O)ₙR⁴ and protected -OH,
where n is 0-2,
R⁴ is hydrogen, alkyl, cycloalkyl, C₁-C₁₂aryl, substituted alkyl, substituted cycloalkyl and substituted C₁-C₁₂aryl, and
R¹⁰ and R¹¹ are independently hydrogen, cycloalkyl, C₁-C₁₂aryl, substituted cycloalkyl, substituted C₁-C₁₂aryl, alkyl or alkyl substituted with one or more substituents selected from the group consisting of: alkoxy, acyloxy, aryloxy, amino, N-acylamino, oxo, hydroxy, -C(O)OR⁴, -S(O)ₙR⁴, -C(O)NR⁴R⁴, -S(O)₂NR⁴R⁴, nitro, cyano, cycloalkyl, substituted cycloalkyl, halogen, aryl, substituted aryl and protected -OH,
or R¹⁰ and R¹¹ taken together with the nitrogen to which they are attached represent a 5 to 6 member saturated ring containing up to one other heteroatom selected from oxygen and nitrogen,
where R⁴ is as described above and n is 0-2;
and/or a pharmaceutically acceptable salt, hydrate, solvate or ester thereof;
provided that at least one of R, R¹, R² and R³ is a substituted aryl group or a heterocyclic methylene substituent as represented in Formula (III), in the manufacture of medicament for the treatment of Nervous system disorders.

2. Use according to claim 1 wherein the compound is represented by the following Formula (II) 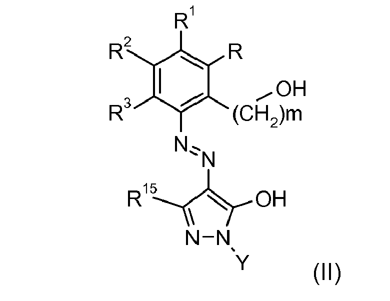 wherein:
R, R¹, R² and R³ are each independently selected from hydrogen, C₁₋₆alkyl, -(CH₂)ₚOR⁴, -C(O)OR⁴, formyl, nitro, cyano, halogen, aryl, substituted aryl, substituted alkyl, -S(O)ₙR⁴, cycloalkyl, -NR⁵R⁶, protected -OH, -CONR⁵R⁶, phosphonic acid, sulfonic acid, phosphinic acid, -SO₂NR⁵R⁶, and a heterocyclic methylene substituent as represented by Formula (III), 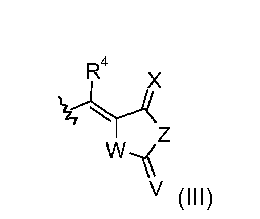 where
p is 0-6,
n is 0-2,
V, W, X and Z are each independently selected from O, S, and NR¹⁶, where R¹⁶ is selected from: hydrogen, alkyl, cycloalkyl, C₁-C₁₂aryl, substituted alkyl, substituted cycloalkyl and
substituted C₁-C₁₂aryl,
R⁴ is hydrogen, alkyl, cycloalkyl, C₁-C₁₂aryl, substituted alkyl, substituted cycloalkyl and substituted C₁-C₁₂aryl, and
R⁵ and R⁶ are each independently selected from hydrogen, alkyl, substituted alkyl, C₃-₆cycloalkyl, and aryl,
or R⁵ and R⁶ taken together with the nitrogen to which they are attached represent a 5 to 6 member saturated ring containing up to one other heteroatom selected from oxygen and nitrogen;
R¹⁵ is selected from the group consisting of alkyl, C₁-C₁₂aryl, hydroxy, alkoxy, substituted alkyl, substituted C₁-C₁₂aryl and halogen;
m is 0-6; and
Y is selected from alkyl, substituted alkyl and a cyclic or polycyclic aromatic ring containing from 3 to 14 carbon atoms and optionally containing from one to three heteroatoms, provided that when the number of carbon atoms is 3 the aromatic ring contains at least two heteroatoms and when the number of carbon atoms is 4 the aromatic ring contains at least one heteroatom, and optionally substituted with one or more substituents selected from the group consisting of: alkyl, substituted alkyl, C₁-C₁₂aryl, substituted cycloalkyl, substituted C₁-C₁₂aryl, hydroxy, aryloxy, alkoxy, cycloalkyl, nitro, cyano, halogen and protected -OH;
and/or a pharmaceutically acceptable salt, hydrate, solvate or ester thereof;
provided that at least one of R, R¹, R² and R³ is a substituted aryl group or a heterocyclic methylene substituent as represented in Formula (III).

3. Use according to claims 1-2 wherein the compound is selected from:
4'-{N'-[1-(3,4-Dimethylphenyl)-3-methyl-5-oxo-1,5-dihydropyrazol-4-ylidene]hydrazino}-3'-hydroxybiphenyl-4-carboxylic acid;
4'-{N'-[1-(3,4-Dimethylphenyl)-3-methyl-5-oxo-1,5-dihydropyrazol-4-ylidene]hydrazino}-3'-hydroxybiphenyl-3-carboxylic acid;
3'-{N'-[1-(3,4-Dimethylphenyl)-3-methyl-5-oxo-1,5-dihydropyrazol-4-ylidene]hydrazino}-2'-hydroxybiphenyl-3-carboxylic acid;
3'-{N'-[1-(4-tert-Butylphenyl)-3-methyl-5-oxo-1,5-dihydropyrazol-4-ylidene]hydrazino}-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-5'-chloro-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-2'-hydroxybiphenyl-3-carboxylic acid;
3-Aza-3'-{N'-[1-(4-tert-butylphenyl)-3-methyl-5-oxo-1,5-dihydropyrazol-4-ylidene]hydrazino}-2'-hydroxybiphenyl-5-carboxylic acid;
2-Aza-5'-chloro-3'-{N'-[1-(3,4-dimethylphenyl)-3-methyl-5-oxo-1,5-dihydropyrazol-4-ylidene]hydrazino}-2'-hydroxybiphenyl-3-carboxylic acid;
2-Aza-3'-{N'-[1-(4-tert-butylphenyl)-3-methyl-5-oxo-1,5-dihydropyrazol-4-ylidene]hydrazino}-2'-hydroxy-5'-methylbiphenyl-3-carboxylic acid;
2-Aza-3'-{N'-[1-(3,4-dimethylphenyl)-3-methyl-5-oxo-1,5-dihydropyrazol-4-ylidene]hydrazino}-2'-hydroxy-5'-methylbiphenyl-3-carboxylic acid;
3'-{N'-[1-(4-tert-Butylphenyl)-3-methyl-5-oxo-1,5-dihydropyrazol-4-ylidene]hydrazino}-2'-hydroxy-5'-methylbiphenyl-3-carboxylic acid;
3-{N'-[1-(3,4-dimethylphenyl)-3-methyl-5-oxo-1,5-dihydropyrazol-4-ylidene]hydrazino}-2-hydroxy-3'-tetrazol-5-ylbiphenyl;
3'-{N'-[1-(3,4-dimethylphenyl)-3-methyl-5-oxo-1,5-dihydropyrazol-4-ylidene]hydrazino}-5'-fluoro-2'-hydroxybiphenyl-3-carboxylic acid;
7-({N'-[1-(3,4-dimethylphenyl)-3-methyl-5-oxo-1,5-dihydropyrazol-4-ylidene]hydrazino}-2-hydroxyphenyl)quinolin-4[1H]-one-3-carboxylic acid;
7-({N'-[1-(4-tert-butylphenyl)-3-methyl-5-oxo-1,5-dihydropyrazol-4-ylidene]hydrazino}-2-hydroxyphenyl)quinolin-4[1H]-one-3-carboxylic acid;
3-Aza-3'-{N'-[1-(3,4-dimethylphenyl)-3-methyl-5-oxo-1,5-dihydropyrazol-4-ylidene]hydrazino}-2'-hydroxybiphenyl-5-carboxylic acid;
3-Aza-3'-(N'-[1-{3-methyl-[4-(1-methylethyl)phenyl]-5-oxo-1,5-dihydropyrazol-4-ylidene}hydrazino)-2'-hydroxybiphenyl-5-carboxylic acid;
3-Aza-3'-{N'-[1-(4-tertbutylphenyl-3-methyl-5-oxo-1,5-dihydropyrazol-4-ylidene]hydrazino}-2'-hydroxybiphenyl-5-carboxylic acid;
5'-Chloro-3'-{N'-[1-(3,4-dimethylphenyl)-3-methyl-5-oxo-1,5-dihydropyrazol-4-ylidene]hydrazino}-2'-hydroxybiphenyl-3-carboxylic acid;
3'-{N'-[1-(3,4-Dimethylphenyl)-3,5-dioxo-1,5-dihydropyrazol-4-ylidene]hydrazino}-2'-hydroxybiphenyl-3-carboxylic acid;
3'-{N'-[1-(2-Ethoxy-2-oxoethyl)-3-methyl-5-oxo-1,5-dihydropyrazol-4-ylidene]hydrazino}-2'-hydroxybiphenyl-3-carboxylic acid;
3-{N'-[1-(3,4-dimethylphenyl)-3-methyl-5-oxo-1,5-dihydropyrazol-4-ylidene]hydrazino}-2-hydroxy-4'-(tetrazol-5-yl)biphenyl;
3'-(N'-{1-[2-(N-tert-butyl)amino-2-oxoethyl]-3-methyl-5-oxo-1,5-dihydropyrazol-4-ylidene}hydrazino)-2'-hydroxybiphenyl-3-carboxylic acid;
3'-{N'-[3-Chloro-1-(3,4-dimethylphenyl)-5-oxo-1,5-dihydropyrazol-4-ylidene]hydrazino}-2'-hydroxybiphenyl-3-carboxylic acid;
5-chloro-3-{N'-[1-(3,4-dimethylphenyl)-3-methyl-5-oxo-1,5-dihydropyrazol-4-ylidene]hydrazino}-2-hydroxy-4'-(tetrazol-5-yl)biphenyl;
3'-{N'-[1-(3,4-Dimethylphenyl)-3-methyl-5-oxo-1,5-dihydropyrazol-4-ylidene]hydrazino}-2'-hydroxybiphenyl-3,5-dicarboxylic acid;
3-Aza-3'-{N'-[1-(3,4-dimethylphenyl)-3-methyl-5-oxo-1,5-dihydropyrazol-4-ylidene]hydrazino}-2'-hydroxy-5'-methylbiphenyl-5-carboxylic acid;
3'-{N'-[1-(3,4-Dimethylphenyl)-3-methyl-5-oxo-1,5-dihydropyrazol-4-ylidene]hydrazino}-2'-hydroxybiphenyl-4-carboxylic acid;
3'-{N'-[1-(3,4-Dimethylphenyl)-3-methoxy-5-oxo-1,5-dihydropyrazol-4-ylidene]hydrazino}-2'-hydroxybiphenyl-3-carboxylic acid;
3'-{N'-[1-(4-methoxyphenyl)-3-methyl-5-oxo-1,5-dihydropyrazol-4-ylidene]hydrazino}-2'-hydroxybiphenyl-3-carboxylic acid;
(3-{N'-[1-(3,4-dimethylphenyl)-3-methyl-5-oxo-1,5-dihydropyrazol-4-ylidene]hydrazino}-2-hydroxy-3'-biphenyl)-1,1,1,-trifluoromethanesulfonamide;
3'-{N'-[1-(3,4-Dichlorophenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(3-trifluoromethylphenyl)-1,5-dihydropyrazol-4-ylidene]hydrazino}-2'-hydroxybiphenyl-3-carboxylic acid;
8-{N'-[1-(3,4-dimethylphenyl)-3-methyl-5-oxo-1,5-dihydropyrazol-4-ylidene]hydrazino}quinolin-4[1H]-one-3-carboxylic acid;
3'-{N'-[3-methyl-5-oxo-1-(4-trifluoromethylphenyl)-1,5-dihydropyrazol-4-ylidene]hydrazino}-2'-hydroxybiphenyl-3-carboxylic acid;
3'-{N'-[3-methyl-5-oxo-1-(4-N-methylcarboxamidolphenyl)-1,5-dihydropyrazol-4-ylidene]hydrazino}-2'-hydroxybiphenyl-3-carboxylic acid;
N-[1-(3'-{N'-[1-(3,4-dimethylphenyl)-3-methyl-5-oxo-1,5-dihydropyrazol-4-ylidene]hydrazino}-2'-hydroxybiphenyl-3-yl)methanoyl]methanesulfonamide;
3'-{N'-[3-methyl-5-oxo-1-phenyl-1,5-dihydropyrazol-4-ylidene]hydrazino}-2'-hydroxybiphenyl-3-carboxylic acid;
3'-{N'-[3-methyl-1-(4-methylphenyl)-5-oxo-1,5-dihydropyrazol-4-ylidene]hydrazino}-2'-hydroxybiphenyl-3-carboxylic acid;
3'-{N'-[1-(4-chlorophenyl)-3-methyl-5-oxo-1,5-dihydropyrazol-4-ylidene]hydrazino}-2'-hydroxybiphenyl-3-carboxylic acid;
3'-{N'-[1-(4-fluorophenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(4-trifluoromethoxyphenyl)-1,5-dihydropyrazol-4-ylidene]hydrazino}-2'-hydroxybiphenyl-3-carboxylic acid;
3'-{N'-[1-(3,4-dimethylphenyl)-3-ethoxy-5-oxo-1,5-dihydropyrazol-4-ylidene]hydrazino}-2'-hydroxybiphenyl-3-carboxylic acid;
3'-{N'-[1-(3,4-dimethylphenyl)-3-(1-methylethoxy)-5-oxo-1,5-dihydropyrazol-4-ylidene]hydrazino}-2'-hydroxybiphenyl-3-carboxylic acid;
3'-{N'-[3-tert-butyl-1-(3,4-dimethylphenyl)-5-oxo-1,5-dihydropyrazol-4-ylidene]hydrazino}-2'-hydroxybiphenyl-3-carboxylic acid;
3'-{N'-[3-methyl-1-(4-methyl-2,3,5,6-tetrafluorophenyl)-5-oxo-1,5-dihydropyrazol-4-ylidene]hydrazino}-2'-hydroxybiphenyl-3-carboxylic acid;
3'-{N'-[1-(4-fluoro-3-methylphenyl)-3-methyl-5-oxo-1,5-dihydropyrazol-4-ylidene]hydrazino}-2'-hydroxybiphenyl-3-carboxylic acid;
3'-{N'-[1-(3.4-dimethylphenyl)-3-phenyl-5-oxo-1,5-dihydropyrazol-4-ylidene]hydrazino}-2'-hydroxybiphenyl-3-carboxylic acid;
3-{N'-[1-(3,4-dimethylphenyl)-5-oxo-3-phenyl-1,5-dihydropyrazol-4-ylidene]hydrazino}-2-hydroxy-3'-tetrazol-5-ylbiphenyl;
3-{N'-[1-(3,4-dimethylphenyl)-3-methoxy-5-oxo-1,5-dihydropyrazol-4-ylidene]hydrazino}-2-hydroxy-3'-tetrazol-5-ylbiphenyl;
3-{N'-[1-(3,4-dimethylphenyl)-3-ethoxy-5-oxo-1,5-dihydropyrazol-4-ylidene]hydrazino}-2-hydroxy-3'-tetrazol-5-ylbiphenyl;
3-{N'-[1-(3,4-dimethylphenyl)-3-(1-methylethoxy)-5-oxo-1,5-dihydropyrazol-4-ylidene]hydrazinol-2-hydroxy-3'-tetrazol-5-ylbiphenyl;
3-{N'-[1-(4-fluorophenyl)-3-methyl-5-oxo-1,5-dihydropyrazol-4-ylidene]hydrazino}-2-hydroxy-3'-tetrazol-5-ylbiphenyl;
3-{N'-[1-(4-fluoro-3-methylphenyl)-3-methyl-5-oxo-1,5-dihydropyrazol-4-ylidene]hydrazino}-2-hydroxy-3'-tetrazol-5-ylbiphenyl;
3-{N'-[3-methyl-5-oxo-1-(4-trifluoromethylphenyl)-1,5-dihydropyrazol-4-ylidene]hydrazino}-2-hydroxy-3'-tetrazol-5-ylbiphenyl;
3'-{N'-[1-(3.4-dimethylphenyl)-3-(pyridin-4-yl-5-oxo-1,5-dihydropyrazol-4-ylidene]hydrazino}-2'-hydroxybiphenyl-3-carboxylic acid;
3-{N'-[1-(3,4-dimethylphenyl)-3-pyridin-4-yl-5-oxo-1,5-dihydropyrazol-4-ylidene]hydrazino}-2-hydroxy-3'-tetrazol-5-ylbiphenyl;
3-{N'-[1-(3,4-dimethylphenyl)-3-pyridin-2-yl-5-oxo-1,5-dihydropyrazol-4-ylidene]hydrazino}-2-hydroxy-3'-tetrazol-5-ylbiphenyl;
3'-{N'-[1-(3.4-dimethylphenyl)-3-(pyridin-2-yl-5-oxo-1,5-dihydropyrazol-4-ylidene]hydrazino}-2'-hydroxybiphenyl-3-carboxylic acid;
3-{N'-[1-(3-fluoro-4methylphenyl)-3-methyl-5-oxo-1,5-dihydropyrazol-4-ylidene]hydrazino}-2-hydroxy-3'-tetrazol-5-ylbiphenyl;
3'-{N'-[1-(3-fluoro-4-methylphenyl)-3-methyl-5-oxo-1,5-dihydropyrazol-4-ylidene]hydrazino}-2'-hydroxybiphenyl-3-carboxylic acid;
3'-{N'-[3-methyl-5-oxo-1-(4-trifluoromethylpyrimidin-2-yl)-1,5-dihydropyrazol-4-ylidene]hydrazino}-2'-hydroxybiphenyl-3-carboxylic acid;
3'-N-tert-butoxycarbonylamino-3-{N'-[1-(3,4-Dimethylphenyl)-3-methyl-5-oxo-1,5-dihydropyrazol-4-ylidene]hydrazino}-2-hydroxybiphenyl;
3'-amino-3-{N'-[1-(3,4-dimethylphenyl)-3-methyl-5-oxo-1,5-dihydropyrazol-4-ylidene]hydrazino}-2-hydroxybiphenyl;
3-{N'-[1-(3-fluorophenyl)-3-methyl-5-oxo-1,5-dihydropyrazol-4-ylidene]hydrazino}-2-hydroxy-3'-tetrazol-5-ylbiphenyl;
3'-{N'-[1-(3-fluorophenyl)-3-methyl-5-oxo-1,5-dihydropyrazol-4-ylidene]hydrazino}-2'-hydroxybiphenyl-3-carboxylic acid;
3-{N'-[3-methyl-5-oxo-1-(2,3,4,5,6-pentafluorophenyl)-1,5-dihydropyrazol-4-ylidene]hydrazino}-2-hydroxy-3'-tetrazol-5-ylbiphenyl;
3'-{N'-[3-methyl-5-oxo-1-(2,3,4,5,6-pentafluorophenyl)-1,5-dihydropyrazol-4-ylidene]hydrazino}-2'-hydroxybiphenyl-3-carboxylic acid;
3'-{N'-[1-(3,4-difluorophenyl)-3-methyl-5-oxo-1,5-dihydropyrazol-4-ylidene]hydrazino}-2'-hydroxybiphenyl-3-carboxylic acid;
3'-{N'-[1-(3,4-dimethyl phenyl)-3-methoxymethyl-5-oxo-1,5-dihydropyrazol-4-ylidene]hydrazino}-2'-hydroxybiphenyl-3-carboxylic acid;
3-{N'-[1-(3,4-dimethylphenyl)-3-methoxymethyl-5-oxo-1,5-dihydropyrazol-4-ylidene]hydrazino}-2-hydroxy-3'-tetrazol-5-ylbiphenyl;
3-{N'-[1-(3,4-difluorophenyl)-3-methyl-5-oxo-1,5-dihydropyrazol-4-ylidene]hydrazino}-2-hydroxy-3'-tetrazol-5-ylbiphenyl;
3'-{N'-[1-(3,4-dimethylphenyl)-5-oxo-3-trifluoromethyl-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-6-fluoro-2'-hydroxybiphenyl-3-carboxylic acid;
3'-{N'-[1-(3,4-dimethylphenyl)-5-oxo-3-propyl-1,5-dihydropyrazol-4-ylidene]hydrazino}-2'-hydroxybiphenyl-3-carboxylic acid;
3-{N'-[1-(3,4-dimethylphenyl)-5-oxo-3-propyl-1,5-dihydropyrazol-4-ylidene]hydrazino}-2-hydroxy-3'-tetrazol-5-ylbiphenyl;
3'-{N'-[1-(3,4-dimethylphenyl)-3-(1-methyl-H-pyrrol-3-yl)-5-oxo-1,5-dihydropyrazol-4-ylidene]hydrazino}-2'-hydroxybiphenyl-3-carboxylic acid;
3-{N'-[1-(3,4-dimethylphenyl)-3-(1-methyl-1H-pyrrol-3-yl)-5-oxo-1,5-dihydropyrazol-4-ylidene]hydrazino}-2-hydroxy-3'-tetrazol-5-ylbiphenyl;
3'-{N'-[1-(3,4-dimethylphenyl)-3-furan-2-yl-5-oxo-1,5-dihydropyrazol-4-ylidene]hydrazino}-2'-hydroxybiphenyl-3-carboxylic acid;
3-{N'-[1-(3,4-dimethylphenyl)-3-furan-2-yl-5-oxo-1,5-dihydropyrazol-4-ylidene]hydrazino}-2-hydroxy-3'-tetrazol-5-ylbiphenyl;
N-(2'-hydroxy-3'-{N'-[3-methyl-5-oxo-1-(4-trifluoromethyl-phenyl)-1,5-dihydro-pyrazol-4-ylidene]hydrazino}biphenyl-3-yl)-1,1,1-trifluoromethanesulfonamide;
N-(2'-hydroxy-3'-{N'-[1-(3-fluoro-4-methylphenyl)-3-methyl-5-oxo-1,5-dihydro-pyrazol-4-ylidene]hydrazino}biphenyl-3-yl)-1,1,1-trifluoromethanesulfonamide;
N-(2'-hydroxy-3'-{N'-[1-(4-fluoro-3-methylphenyl)-3-methyl-5-oxo-1,5-d i hyd ro-pyrazol-4-ylidene]hydrazino}biphenyl-3-yl)-1,1,1-trifluoromethanesulfonamide;
N-(2'-hydroxy-3'-{N'-[1-(3,4-difluorophenyl)-3-methyl-5-oxo-1,5-dihydro-pyrazol-4-ylidene]hydrazino}biphenyl-3-yl)-1,1,1-trifluoromethanesulfonamide;
N-(3'-{N'-[1-(3,4-dimethylphenyl)-3-methyl-5-oxo-1,5-dihydropyrazol-4-ylidene]hydrazino}-2'-hydroxybiphenyl-3-yl)guanidine;
3'-{N'-[1-(3,4-dimethylphenyl)-3-ethyl-5-oxo-1,5-dihydropyrazol-4-ylidene]hydrazino}-2'-hydroxybiphenyl-3-carboxylic acid;
3-{N'-[1-(3,4-dimethylphenyl)-3-ethyl-5-oxo-1,5-dihydropyrazol-4-ylidene]hydrazino}-2-hydroxy-3'-tetrazol-5-ylbiphenyl;
3'-{N'-[1-(3,4-dimethylphenyl)-5-oxo-3-thien-2-yl-1,5-dihydropyrazol-4-ylidene]hydrazino}-2'-hydroxybiphenyl-3-carboxylic acid;
3'-{N'-[3-cyclopropyl-1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-5-oxo-3-thiazol-2-yl-1,5-dihydropyrazol-4-ylidene]hydrazino}-2'-hydroxybiphenyl-3-carboxylic acid;
3'-{N'-[1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-3-(1-methylethyl)-5-oxo-1,5-dihydropyrazol-4-ylidene]hydrazino}-2'-hydroxybiphenyl-3-carboxylic acid;
3'-{N'-[3-(benzyloxymethyl)-1-(3,4-dimethylphenyl)-5-oxo-1,5-dihydropyrazol-4-ylidene]hydrazino}-2'-hydroxybiphenyl-3-carboxylic acid;
3'-{N'-[3-ethyl-5-oxo-1-(4-trifluoromethylphenyl)-1,5-dihydropyrazol-4-ylidene]hydrazino}-2'-hydroxybiphenyl-3-carboxylic acid;
3'-{N'-[5-oxo-1-(4-trifluoromethylphenyl)-1,5-dihydropyrazol-4-ylidene]hydrazino}-2'-hydroxybiphenyl-3-carboxylic acid;
3'-{N'-[-1-(3,4-dimethylphenyl)-3-hydroxymethyl-5-oxo-1,5-dihydropyrazol-4-ylidene]hydrazino}-2'-hydroxybiphenyl-3-carboxylic acid;
3'-{N'-[3-benzyloxymethyl-5-oxo-1-(4-trifluoromethylphenyl)-1,5-dihydropyrazol-4-ylidene]hydrazino}-2'-hydroxybiphenyl-3-carboxylic acid;
3'-{N'-[-1-(3,4-dimethylphenyl)-3-methylsulfanylmethyl-5-oxo-1,5-dihydropyrazol-4-ylidene]hydrazino}-2'-hydroxybiphenyl-3-carboxylic acid;
3'-{N'-[-1-(3,4-dimethylphenyl)-5-oxo-3-thiophen-3-yl-1,5-dihydropyrazol-4-ylidene]hydrazino}-2'-hydroxybiphenyl-3-carboxylic acid;
3'-{N'-[5-oxo-1-(4-trifluoromethylphenyl)-3-thiophen-3-yl-1,5-dihydropyrazol-4-ylidene]hydrazino}-2'-hydroxybiphenyl-3-carboxylic acid;
3'-{N'-[5-oxo-1-(4-trifluoromethylphenyl)-3-methylsulfanylmethyl-1,5-dihydropyrazol-4-ylidene]hydrazino}-2'-hydroxybiphenyl-3-carboxylic acid;
N-(3'-{N'-[1-(3,4-dimethylphenyl)-3-methyl-5-oxo-1,5-dihydro-pyrazol-4-ylidene]hydrazino}-2'-hydroxybiphenyl-3-yl)methanesulfonamide;
3'-[N'-(1-benzo[1,3]dioxol-5-yl-3-methyl-5-oxo-1,5-dihydropyrazol-4-ylidene)hydrazino]-2'-hydroxybiphenyl-3-carboxylic acid;
3'-{N'-[1-(3,5-dimethylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-4'-hydroxybiphenyl-4-carboxylic acid;
3'-{N'-[1-(3-chloro-4-methylphenyl)-3-methyl-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-4'-hydroxybiphenyl-3-carboxylic acid;
3'-{N'-[1-(3,4-dimethylphenyl)-3-methyl-5-oxo-1,5-dihydropyrazol-4-ylidene]hydrazino}-2'-hydroxybiphenyl-3-phosphonic acid;
3'-{N'-[1-(3,4-dimethylphenyl)-3-methyl-5-oxo-1,5-dihydropyrazol-4-ylidene]hydrazino}-2'-hydroxybiphenyl-3,4-dicarboxylic acid;
2',6-dihydroxy-3'-{N'-[1-(3,4-dimethylphenyl)-3-methyl-5-oxo-1,5-dihydropyrazol-4-ylidene]hydrazino}biphenyl-3-carboxylic acid;
4-aza-3'-{N'-[1-(3,4-dimethylphenyl)-3-methyl-5-oxo-1,5-dihydropyrazol-4-ylidene]hydrazino}-2'-hydroxybiphenyl-5-carboxylic acid;
3'-{N'-[1-(3,4-dimethylphenyl)-5-oxo-1,5-dihydropyrazol-4-ylidene]hydrazino}-2'-hydroxybiphenyl-3-carboxylic acid;
3'-{N'-[1-(3,4-dimethylphenyl)-3-methyl-5-oxo-1,5-dihydropyrazol-4-ylidene]hydrazino}-2'-hydroxybiphenyl-3-sulfonic acid; and
5-(3'-{N'-[1-(3,4-Dimethylphenyl)-3-methyl-5-oxo-1,5-dihydropyrazol-4-ylidene]hydrazino}-2'-hydroxybiphenyl-3-ylmethylene)thiazolidine-2,4-dione;
and/or a pharmaceutically acceptable salt, hydrate, solvate or ester thereof.

4. Use according to claim 3 wherein the compound is:
3'-{N'-[1-(3,4-Dimethylphenyl)-3-methyl-5-oxo-1,5-dihydropyrazol-4-ylidene]hydrazino}-2'-hydroxybiphenyl-3-carboxylic acid, or a pharmaceutically acceptable salt thereof.

5. Use according to claims 1-4 wherein the compound is administered orally.

6. Use according to claims 1-4 wherein the compound is administered parenterally.

7. Use according to any of claims 1-6 wherein then nervous system disorder is selected from transverse myelitis, multiple sclerosis, demyelination occurring after trauma to the brain or spinal cord, acute brain injury, head trauma, spinal cord injury, peripheral nerve injury, ischaemic brain injury, hereditary myelin disorder of the CNS, epilepsy, perinatal asphyxia, asphyxia, anoxia, status epilepticus, and stroke..

8. Use according to any proceeding claim wherein the medicament further comprises a therapeutically effective amount of an agent selected from the group consisting of: a colony stimulating factor, cytokine, chemokine, interleukin or cytokine receptor agonist or antagonists, soluble receptors, receptor agonists or antagonist antibodies, or small molecules or peptides that act by the same mechanisms one or more of said agents.

9. Use according to claim 8 wherein the agent is selected from the group consisting of:
G-CSF, GM-CSF, TPO, M-CSF, EPO, Gro-beta, IL-11, SCF, FLT3 ligand, LIF, IL-3, IL-6, IL-1, Progenipoietin, NESP, SD-01, IL-8, or IL-5 or a biologically active derivative of any of said agents.

10. A pharmaceutical composition for use in the treatment of nervous system disorders in mammals comprising a non-peptide TPO receptor agonist according to claims 1-4.
